(19) Europäisches Patentamt
European Patent Office
Office européen des brevets

(11) **EP 4 054 412 B1**

(12) **EUROPEAN PATENT SPECIFICATION**

(45) Date of publication and mention of the grant of the patent:
**06.03.2024 Bulletin 2024/10**

(21) Application number: **20807096.1**

(22) Date of filing: **03.11.2020**

(51) International Patent Classification (IPC):
***A61B 5/08*** ***(2006.01)*** ***A61B 5/097*** ***(2006.01)***
***G01N 33/497*** ***(2006.01)***

(52) Cooperative Patent Classification (CPC):
**A61B 5/082;** A61B 5/097; G01N 33/497;
G01N 2800/122

(86) International application number:
**PCT/GB2020/052778**

(87) International publication number:
**WO 2021/089992 (14.05.2021 Gazette 2021/19)**

# (54) EXHALED BREATH COLLECTION DEVICE

AUSATEMGERÄT FÜR ATEMLUFT

DISPOSITIF DE COLLECTE D'AIR EXPIRÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.11.2019 GB 201916214**

(43) Date of publication of application:
**14.09.2022 Bulletin 2022/37**

(73) Proprietors:
- **Chen, Yu-Chih**
  **London SW11 7AY (GB)**
- **O'Hare, Danny**
  **East Sussex BN1 4JY (GB)**

(72) Inventors:
- **Chen, Yu-Chih**
  **London SW11 7AY (GB)**
- **O'Hare, Danny**
  **East Sussex BN1 4JY (GB)**

(74) Representative: **J A Kemp LLP**
**80 Turnmill Street**
**London EC1M 5QU (GB)**

(56) References cited:
**WO-A1-2008/022183** **WO-A1-2018/172761**
**US-A1- 2008 183 094** **US-A1- 2008 221 806**

- **YU-CHIH CHEN ET AL: "Exhaled breath condensate based breath analyser - a disposable hydrogen peroxide sensor and smart analyser", ANALYST, vol. 145, no. 10, 14 April 2020 (2020-04-14), pages 3549-3556, XP055768004, UK ISSN: 0003-2654, DOI: 10.1039/C9AN02438G**
- **JIN RONGRONG ET AL: "Layered double hydroxide supported Prussian blue nanocomposites for electrocatalytic reduction of H2O2", ANALYTICAL METHODS, vol. 4, no. 9, 7 June 2012 (2012-06-07), page 2704, XP055786329, GBR ISSN: 1759-9660, DOI: 10.1039/c2ay25306b Retrieved from the Internet: URL:https://pubs.rsc.org/tr/content/articlepdf/2012/ay/c2ay25306b>**
- **WANG JUNJIE ET AL: "Enzymeless voltammetric hydrogen peroxide sensor based on the use of PEDOT doped with Prussian Blue nanoparticles", MIKROCHIMICA ACTA, SPRINGER VERLAG, VIENNA, AT, vol. 184, no. 2, 5 December 2016 (2016-12-05), pages 483-489, XP036143081, ISSN: 0026-3672, DOI: 10.1007/S00604-016-2025-Y [retrieved on 2016-12-05]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 054 412 B1

- Hakimi Mahdieh ET AL: "Experimental Study on PEDOT:PSS Conductive Polymer and N-doped Graphene Quantum Dots for H2O2 Sensing", Bulletin de la Société Royale des Sciences de Liège, vol. 85 1 June 2016 (2016-06-01), pages 261-268, XP055786783, Retrieved from the Internet: URL:https://popups.uliege.be/0037-9565/index.php?id=5327&file=1 [retrieved on 2021-03-17]
- ZAMURUYEV KONSTANTIN O ET AL: "Effect of temperature control on the metabolite content in exhaled breath condensate", ANALYTICA CHIMICA ACTA, ELSEVIER, AMSTERDAM, NL, vol. 1006, 30 December 2017 (2017-12-30), pages 49-60, XP086136070, ISSN: 0003-2670, DOI: 10.1016/J.ACA.2017.12.025 [retrieved on 2017-12-30]
- GOLDONI MATTEO ET AL: "Influence of condensation temperature on selected exhaled breath parameters", BMC PULMONARY MEDICINE, BIOMED CENTRAL, LONDON, GB, vol. 5, no. 1, 1 September 2005 (2005-09-01), page 10, XP021005501, ISSN: 1471-2466, DOI: 10.1186/1471-2466-5-10

## Description

The present invention relates to an exhaled breath collection device. More particularly, the device disclosed herein is suitable for collection and measurement of a biomarker in exhaled breath.

Exhaled breath collection devices are used in a variety of applications including in the diagnosis, monitoring and treatment of illnesses such as chronic obstructive pulmonary disease (COPD). Various analytes, for example biomarkers such as glucose, which can be indicative of diabetes, and hydrogen peroxide ($H_2O_2$), which can give an indication of oxidative stress, may be present in the exhaled breath. It is therefore useful to have some means to collect samples of exhaled breath and to analyse the samples to measure the biomarkers. There is often a relatively low concentration of the biomarker of interest in the exhaled breath samples. Due in part to this low concentration of analyte in exhaled breath samples, most analyses of the collected samples must be carried out in laboratories, with the use of benchtop machines such as fluorometers and mass spectrometers. The use of dedicated laboratory equipment and expertise in the analysis of biomarkers in exhaled breath samples is expensive and time consuming.

Many exhaled breath related medical devices are therefore solely dedicated to sample collection, and do not perform sample analysis because most exhaled breath samples are analysed in laboratories. In that respect, exhaled breath collection collectors have traditionally been designed to collect as large a volume of sample as possible in minimal user operation time.

During sample collection, it can be desirable to condense the sample into a liquid. Some devices have achieved this by making use of passive cooling. That is, the device itself is first cooled for an extended period before it is used to collect a sample. This requirement impedes users from collecting samples for analysis at home. Devices having built-in active cooling have therefore been developed. These exhaled breath collection devices typically adopt thermoelectric cooling devices such as Peltier to condense the exhaled breath and collect the exhaled breath condensate (EBC) samples. Given the requirements to achieve very low temperatures, this approach has led to the development of bulky devices which are not easily portable or suitable for handheld operation. Traditionally, these devices have been expensive and often require operation by trained professionals.

In order to address the above described problems, the present invention is directed to providing a relatively cheap, portable device to collect and analyse exhaled breath in a short period of time. WO 2018/172761 A1 discloses a breath-condensate device which may be used to detect hydrogen peroxide; the device comprises a condensing zone configured to be cooled by a Peltier device; the cooling temperature may be set relative to ambient temperature and may be around 10°C; a volume of condensate collected may be determined by monitoring the power necessary to maintain the cooling temperature. US 2008/183094 Al discloses a method and device for recovering and analyzing respiratory condensates; parameters of the respiratory condensate such as the hydrogen peroxide concentration may be determined. WO 2008/022183 A1 discloses a condensate glucose analyzer. Goldoni Matted Et Al: "Influence of condensation temperature or selected exhaled breath parameters", BMC Pulmonary Medicine, vol. 5, no. 1, 1 September 2005 (2005-09-01), discloses an investigation into effects of changes in cooling temperature on biomarker levels in exhaled breath condensate.

The present invention is defined by the independent claims. Further aspects and preferred embodiments are defined in the dependent claims. Any aspects, embodiments and examples of the present disclosure which do not fall under the scope of the appended claims do not form part of the invention and are merely provided for illustrative purposes.

In another aspect of the present disclosure, the control unit may be configured to control the cooling device such that the temperature remains above 0 °C until the target temperature is reached.

In another aspect of the present disclosure, the cooling device may be configured to reduce the temperature of exhaled breath such that exhaled breath condensate is formed, and the sensor unit may be configured to measure the biomarker in the exhaled breath condensate.

In another aspect, the temperature control unit can, in use, control the cooling device such that the target temperature is variable.

In another aspect, the temperature control unit may be configured such that the target temperature corresponds to a selected biomarker.

In another aspect of the present disclosure, the temperature control unit may be configured to determine the target temperature based on the predetermined value of Henry's law constant corresponding to the selected biomarker.

In another aspect of the present disclosure, the control unit may be configured to set the target temperature using an algorithm according to which a higher target temperature is set for a higher value of Henry's law constant.

In another aspect, the exhaled breath collection device may further comprise a condensation surface configured to be cooled by the cooling device and disposed to be exposed to exhaled breath introduced to the device.

In another aspect, the condensation surface may be disposed in contact with the cooling device. Alternatively, the condensation surface may be a surface of the cooling device.

The exhaled breath collection device further comprises a mouthpiece configured to direct exhaled breath towards the cooling device.

In another aspect, the condensation surface may be disposed perpendicular to the direction of flow of exhaled breath through the mouthpiece.

In another aspect, the sensor unit may be disposed such that exhaled breath condensate formed on the condensation surface is deposited on the sensor unit.

In another aspect, the sensor unit may comprise a biosensor, wherein the biosensor is an electrochemical sensor.

In another aspect, the sensor unit may comprise an inert adsorption layer. The inert adsorption layer may be configured to direct exhaled breath condensate to the biosensor. The inert adsorption layer may comprise one or more porous polymers, or one or more porous ceramics.

In another aspect, the exhaled breath collection device may be configured such that the sensor unit can be removed.

In another aspect, the exhaled breath collection device may further comprise an electrical contact configured to interface with a corresponding electrical contact of the sensor unit.

In another aspect, the exhaled breath collection device may further comprise means to analyse the biomarker measurements taken by the sensor unit.

In another aspect, the sensor unit may comprises an electrode coated with an active agent dispersed in a conductive polymer. The active agent may be potassium ferric ferrocyanide. The conductive polymer may be poly(3,4-ethylenedioxythiophene)-poly(styrenesulfonate).

In an aspect of the disclosure, there is at least 0.1 nmol/cm$^2$ of potassium ferric ferrocyanide on the surface of the electrode.

The exhaled breath collection device further comprises a breath temperature sensor configured to measure the breath temperature. The breath temperature sensor is disposed downstream of the mouthpiece unit in a direction of flow of breath and upstream of the cooling device in a direction of flow of breath.

The exhaled breath collection device further comprises a flow sensor configured to measure the breath flow rate. The flow sensor is disposed downstream of the mouthpiece unit in a direction of flow of breath and upstream of the cooling device in a direction of flow of breath.

The temperature control unit is configured to calculate the specific humidity upstream of the cooling device in a direction of flow of breath using breath temperature measured by the breath temperature sensor, calculate the specific humidity at the exhaust using exhaust temperature measured by the exit temperature sensor, and determine the amount of condensate collected in the device using the specific humidity of breath, the specific humidity at the exhaust and the breath flow rate using flow rate measured by the flow sensor.

In another aspect, the temperature control unit may be configured to calculate the mass transfer coefficient from the breath flow rate measured by the breath flow sensor, the Henry's law constant for the selected biomarker, and temperature of the cooling device; and determined the concentration of analyte collected based on the mass transfer coefficient and the mass of condensate collecte In another aspect, the temperature control unit may be configured to determine a corrected amount of condensate based on the determined amount of condensate collected in the device and a correction factor CF. The correction factor CF based on the ratio of the determined amount of condensate collected in the device to a standardised amount of condensate based on predetermined values of breath temperature downstream of the mouthpiece unit and upstream of the cooling device, breath flow rate downstream of the mouthpiece unit and upstream of the cooling device, temperature of the cooling device and exhaust breath temperature.

The correction factor CF may defined by

$$(1)\qquad CF = \frac{\left(1-\exp\left(\frac{-\overline{k_{overall,m}}\times A}{V_{breath,m}}\right)\right)(\omega_{in,s}-\omega_{out,s})T_{breath,s}}{\left(1-\exp\left(\frac{-\overline{k_{overall,s}}\times A}{V_{breath,s}}\right)\right)(\omega_{in,m}-\omega_{out,m})T_{breath,m}}$$

wherein A is the area of the condensation surface, k(overall,m) is the overall mass transfer coefficient; V(breath,m) is the breath flow rate using flow rate measured by the flow sensor; co(in,m) is the specific humidity upstream of the cooling device; ω(out,m) is the specific humidity at the exhaust; T(breath,m) is the exhaust temperature; k(overall,s) is a predetermined standardised overall mass transfer coefficient; V(breath,s) is a predetermined standardised breath flow rate; ω(in,s) is a predetermined standardised upstream specific humidity; ω(out,s) is a predetermined standardised exhaust specific humidity; and T(breath,s) is a predetermined standardised exhaust temperature.

Aspects of the present disclosure will now be described with reference to the accompanying figures:

**Figure 1 -** Overview of exemplary device configuration.
**Figure 2** - Exemplary temperature control system.

**Figure 3** - Exemplary geometry for impinging flow of breath.
**Figure 4** - Exemplary sensor unit.
**Figure 5** - Exemplary configuration of the device with removable sensor unit.
**Figure 6** - Exemplary breath flow connector plate.
**Figure 7** - Exemplary breath exhaust.
**Figure 8** - Overview of exemplary process of using the device.
**Figure 9** - Overview of exemplary device component interaction.
**Figure 10** - Artificial lung experimental setup
**Figure 11** - Concentration of hydrogen peroxide in the condensate collected against temperature of Peltier device.
**Figure 12** - Exemplary overview of process to set the target temperature according to the value of Henry's law constant associated with the selected biomarker/analyte.
**Figure 13** - Overview of breath flow properties through the device.
**Figure 14** - Exemplary overview of process to determine the amount of condensate collected.
**Figure 15** - Exemplary overview of process to determine the concentration of analyte collected.

Conventional exhaled breath condensate (EBC) collection systems, particularly those directed to chronic obstructive pulmonary disease (COPD) applications, typically apply cooling to a target temperature of 0°C or less, commonly cooling to as low as -20°C, in an effort to collect as much condensate as possible. However, it has been shown that, with the use of different devices to collect and analyse the EBC samples, there have been varying results in the concentration of, for example, hydrogen peroxide which has been measured in the EBC collected from healthy control subjects. Previous research has focused on factors such as patient breathing patterns to account for these discrepancies in analyte concentration. However, the present inventors have discovered that the exhaled breath collection device itself could play a significant role in the reported analyte concentration variations. Specifically, the inventors have identified that a higher concentration of the analyte may be collected by cooling to a target temperature above 0°C.

The process of EBC condensation could be considered as comprising two aspects:

1. Condensation of water into condensate, which would be heat transfer limited as water has a high vapour pressure.
2. Mass transfer of analytes into water condensate.

In terms of condensation, more water (volume) would be condensed if more heat were removed from breath. Therefore, a greater volume of condensate can be obtained with lower cooling temperature. For example, assuming same geometry and identical breath, a cooling surface with -20°C would collect higher volume of EBC than with a 0°C cooling surface. For this reason, most methods of EBC collection in the prior art are directed to obtaining a larger volume of EBC by applying cooling temperatures to reach a target temperature below 0°C.

In terms of mass transfer of analyte into EBC, the dominating mass transfer resistance could be in either:

1. Gas phase mass transfer
2. Liquid phase mass transfer

The resistance is dependent on the analyte, in other words the biomarker to be analysed. In particular this will depend on the diffusion coefficient of the analyte in the gas and liquid phases as well as gas/liquid interphase diffusion, according to Henry's law. For example, in many COPD applications the analyte of interest may be hydrogen peroxide ($H_2O_2$). For hydrogen peroxide, the dominating resistance would be gas phase mass transfer because of thin EBC thickness and high Henry's law constant. This means that the absolute amount, in moles, of hydrogen peroxide in the exhaled breath condensate would in practice be consistent regardless of the cooling surface temperature. Therefore, by using the collection device at a lower temperature the hydrogen peroxide would be more diluted in the EBC and vice versa. Thus, to obtain a higher concentration of hydrogen peroxide in the EBC it is preferable to apply a cooling temperature which is relatively high.

An exhaled breath collection device (1000) of the present disclosure, is configured such that in use it can reach a target temperature greater than 0 °C and less than or equal to 30 °C. Thus, a relatively high concentration of analyte may be collected without requiring a bulky cooling device.

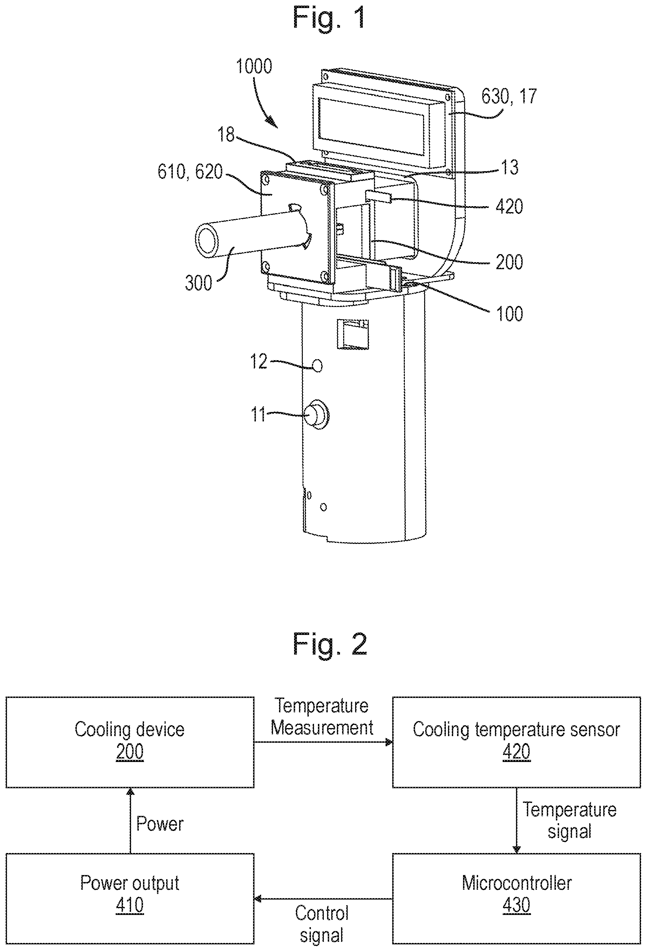

An example exhaled breath collection device (1000), as shown in Fig. 1, may comprise a sensor unit (100) configured to measure a biomarker in exhaled breath, a cooling device (200) configured to reduce a temperature of exhaled breath, and a temperature control unit. The temperature control unit may be configured to control the cooling device (200) to reach a target temperature. The cooling device (200) is configured to reduce the temperature of exhaled breath such that exhaled breath condensate may be formed. The sensor unit (100) is configured to measure the biomarker in the exhaled breath condensate.

As illustrated in figure 1, the exhaled breath collection device (1000) may comprise a power button (11) to enable a user to activate the device. The device may further comprise a mouthpiece (300) configured to direct exhaled breath towards the cooling device (200). The device may also comprise a notification unit (12), such as an LED and/or buzzer, configured to provide a notification when the device is ready for breath collection. The notification unit (12) may be configured to provide a notification when the cooling device (200) has reached the target temperature, as measured by a cooling temperature sensor (420). A breath flow rate indicator (630) may provide the user with information on breath flow rate, according to measurements from a breath flow rate sensor.

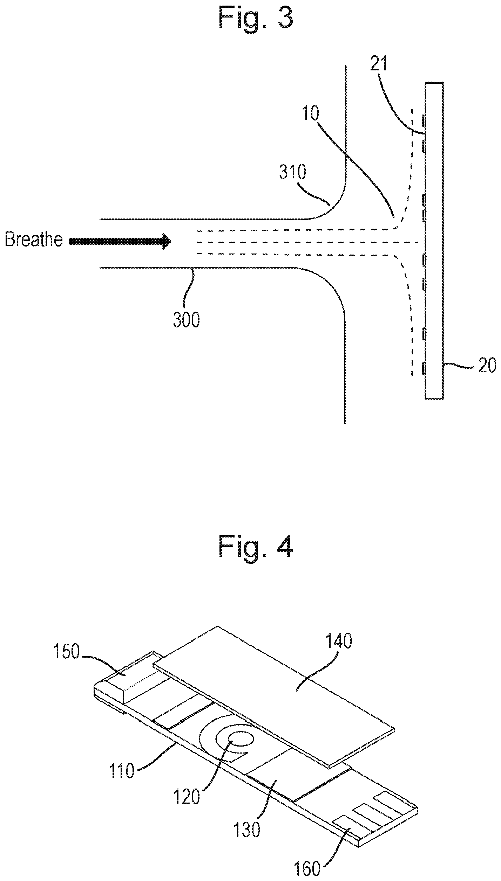

The exhaled breath collection device (1000) may comprise a condensation surface (21) (shown in Fig. 3) configured to be cooled by the cooling device (200) and disposed to be exposed to exhaled breath introduced through the mouthpiece (300). The device may comprise a heat sink (13) to dissipate heat away from the condensation surface (21).

The notification unit (12) may be configured to provide a notification once a sufficient sample has been collected, as detected by the sensor unit (100). A results display unit (17) may be provided to display the results. The device may comprise means to export the results via Bluetooth and/or a USB or alternative connection for further review on a computer. The exhaled breath collection device (1000) is preferably configured to be portable, and more preferably to be handheld during use.

In an exhaled breath collection device (1000) of the present disclosure, the temperature control unit may be configured such that it can, in use, control the cooling device (200) to reach a target temperature greater than 0 °C and less than or equal to 30 °C. Alternatively, the target temperature may be greater than 5 °C and less than or equal to 30 °C, or the target temperature may be greater than 10 °C and less than or equal to 30 °C. Alternatively, the target temperature may be greater than 5 °C and less than or equal to 20 °C, or the target temperature may be greater than 10 °C and less than or equal to 20 °C. A target temperature in the range of greater than or equal to 15 °C and less than or equal to 30 °C is particularly appropriate when the biomarker the sensor unit (100) is configured to measure is hydrogen peroxide.

The control unit may be configured to control the cooling device (200) such that the temperature remains above 0 °C until the target temperature is reached. Preferably, the control unit may be configured to control the cooling device (200) such that the temperature remains above 0 °C until the target temperature is reached.

The condensation surface (21) may be formed of a ceramic based substrate. Alternatively, the condensation surface (21) may be formed of a plastic based substrate, for example high density polyethylene (HDPE), polyethylene terephthalate (PET), polycarbonate (PC), or polysulfone (PS).

The specific target temperature selected within the ranges mentioned above may depend on the material of the condensation surface. For example, with a plastic based substrate, the condensation process may be slower than if the condensation surface (21) is formed of a ceramic based substrate. As a result, it may be preferable to set a target temperature, for example, approximately 5 °C lower for the same biomarker if the condensation surface (21) is formed of a plastic based substrate, compared to if it is formed of a ceramic based substrate.

The exhaled breath collection device (1000) may be suitable for the measurement of biomarkers associated with COPD. More particularly, the exhaled breath collection device (1000) may be configured to measure biomarkers associated with oxidative stress. For example, the exhaled breath collection device (1000) may be configured to measure hydrogen peroxide.

The temperature control unit may be configured to control the cooling device (200) such that the target temperature is maintained for a set time period. The set time period may be between 1 minutes and 10 minutes, or may be between 2 minutes and 10 minutes.

The device is preferably portable. The device may be suitable for handheld use.

*Device Geometry*

The exhaled breath collection device (1000) may comprise a condensation surface (21) configured to be cooled by the cooling device (200) and disposed to be exposed to exhaled breath introduced through the mouthpiece (300). The condensation surface (21) is preferably disposed perpendicular to the direction of flow of exhaled breath through the mouthpiece (300). The condensation surface (21) may be a surface of the cooling device (200). The condensation surface (21) may be a surface of a condensation wall (20), where the condensation wall (20) is an internal wall of the device. The condensation wall (20) is preferably disposed parallel to a surface of the cooling device (200). The condensation wall (20) may be in contact with the cooling device (200). Alternatively, the condensation wall (20) may be adjacent to, but not in contact with, the cooling device (200). Preferably, the condensation surface (21) is flat.

The mouthpiece (300) may comprise a nozzle (310) at a terminal end of the mouthpiece (300) in a direction of flow of breath through the mouthpiece (300). Exhaled breath leaving the mouthpiece (300) enters a flow space (10). The flow space (10) may be defined by internal walls of the device. As illustrated in figure 3, the condensation surface (21) is preferably disposed opposite to the nozzle (310) and perpendicular to the direction of flow of breath through the mouthpiece (300). That is, the breath preferably impinges on the condensation surface substantially perpendicularly.

The exhaled breath leaving the mouthpiece (300) through the nozzle (310) is therefore directed towards the condensation surface (21). The exhaled breath may therefore have impinging flow in the flow space (10). Impinging flow promotes heat and mass transfer resulting in a shorter collection time compared to devices not having a configuration which encourages impinging flow (e.g. a condensation surface substantially parallel to the flow direction).

The internal diameter of the nozzle (310) is preferably greater than or equal to 0.2 times the vertical height of the condensation surface (21) and less than or equal to 0.5 times the breadth of the condensation surface (21).

The distance between the nozzle (310) and the condensation surface (21) is greater than or equal to 0.2 times the breadth of the condensation surface (21) and less than or equal to 1.2 times the height of the condensation surface (21). Wherein the condensation surface (21) may be square. Alternatively, the condensation surface (21) may be rectangular, and the breadth of the condensation surface (21) is the dimension of the shortest side of the condensation surface (21). Alternatively, the condensation surface (21) may be circular, in which case the breadth is the diameter of the condensation surface (21).

The condensation surface (21) may have a breadth of between 20 $mm^2$ and 100 $mm^2$. The condensation surface (21) may have a length of between 20 $mm^2$ and 100 $mm^2$. This size of condensation surface (21) is preferable because it is large enough for sufficient sample quantities to be collected yet small enough that the device is easily portable.

The cooling device (200) may be a Peltier device. Preferably, the cooling device (200) is not curved. Preferably, the cooling device (200) is flat. The arrangement disclosed herein improves exhaled breath collection so that expensive curved surfaces are not required. This is because, with the present configuration, the cooling device does not have to be in close proximity to a portion of the mouthpiece.

*Sensor Architecture*

The sensor unit (100) is preferably disposed in the exhaled breath collection device (1000) such that, during use, exhaled breath condensate formed on the condensation surface (21) is deposited on the sensor unit (100). As illustrated in figure 4, the sensor unit (100) can comprise a biosensor (110) , wherein the biosensor (110) may be an electrochemical sensor. The biosensor (110) may disposed on a front surface (130) of the sensor unit (100). During use, the sensor unit (100) and device may be configured such that the front surface (130) of the sensor unit (100) is disposed in contact with the condensation surface (21). The sensor unit (100) is preferably disposed such that the front surface (130) is in contact with the condensation surface (21) and/or below the condensation surface (21). The front surface (130) of the sensor unit (100) may be parallel to the condensation surface (21) and disposed facing the condensation surface (21). Alternatively, the front surface (130) of the sensor unit (100) may be perpendicular to the condensation surface (21) and may also be below the condensation surface (21) such that condensate formed on the condensation surface (21) is directed to the front surface (130) of the sensor unit (100).

The sensor unit (100) may comprise an inert adsorption layer (140). The inert adsorption layer (140) is preferably disposed covering the biosensor (110). The inert adsorption layer (140) may be adhesively attached to the biosensor (110). A surface of the inert adsorption later may be the front surface (130) of the sensor unit (100). The inert adsorption layer (140) is configured to direct exhaled breath condensate to the biosensor (110). During use, the sensor unit (100) and device may be configured such that the inert adsorption layer (140) is disposed in contact with the condensation surface (21). Alternatively, the sensor unit (100) may be disposed such that the inert adsorption layer (140) is below the condensation surface (21). In either case, the inert adsorption layer (140) is disposed such that condensate formed on the condensation surface (21) is directed to the inert adsorption layer (140). The inert adsorption layer (140) is configured to adsorb condensate such that the condensate is directed to the biosensor (110) on the front surface (130) of the sensor unit (100).

The inert adsorption layer (140) may comprise one or more porous polymers. The inert adsorption layer (140) may consist of one or more porous polymers. Preferably, the one or more porous polymers may be one or more of Polypropylene (PP), polyethylene terephthalate (PET), high-density polyethylene (HDPE), polycarbonates (PC) and polysulfone. The inert adsorption layer (140) may comprise one or more porous ceramics. The inert adsorption layer (140) may consist of one or more porous ceramics. Preferably, the one or more porous ceramics are one or more of porous silica and porous alumina.

The exhaled breath collection device (1000) may further comprise a device electrical contact (16) configured to interface with a corresponding sensor electrical contact (160) of the sensor unit (100). The exhaled breath collection device (1000) according to any of the preceding claims may further comprise means to analyse the biomarker measurements taken by the sensor unit (100). The exhaled breath collection device (1000) may comprise a potentiostat configured to drive the sensor measurement. The electrical contact between the sensor unit (100) and the exhaled breath collection device (1000) enable the device to analyse the results of the biomarker sample measurement from the biosensor (110). The exhaled breath collection device (1000) may further comprise means to display results of the biomarker analysis. It is advantageous that the sample collection, measurements and display of analysed results may be provided in a single device. This reduces the costs associated with purchasing multiple different devices for the different steps in the process. Furthermore, it reduces the time taken to collect and analyse the samples, particularly compared to devices requiring freezing for several hours or requiring samples to be sent to a laboratory for analysis on benchtop machines.

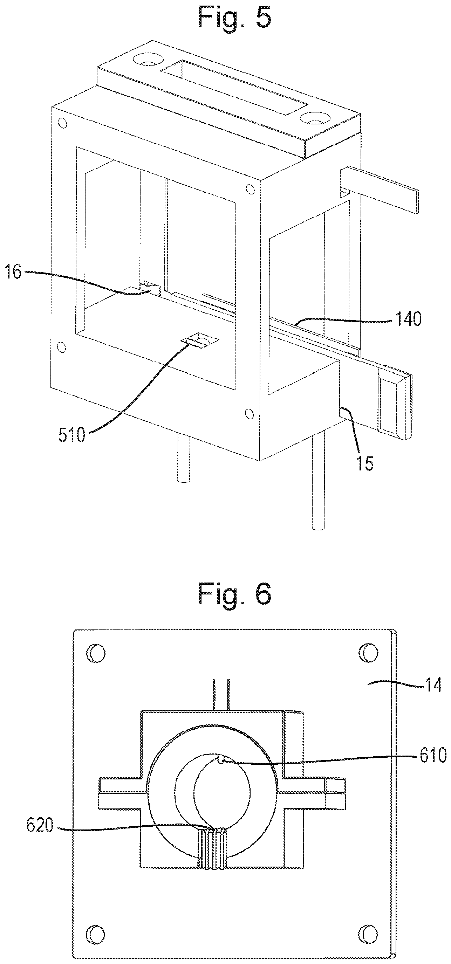

As illustrated in figure 5, the exhaled breath collection device (1000) may be configured such that the sensor unit (100) is removable. With a removable sensor unit (100), it is possible to collect and analyse an exhaled breath sample, then remove the used sensor unit (100) and replace it with a clean sensor for subsequent use of the device. This avoids contamination of the next sample to be collected and analysed by any residue of the previous sample.

The exhaled breath collection device (1000) may comprise a sensor slot (15) wherein the sensor unit (100) is insertable in, and removable from, the sensor slot (15). The sensor unit (100) may comprise a handle (150) configured to protrude from the exhaled breath collection device (1000). The handle (150) enables the sensor unit (100) to be easily inserted and removed from the device. The handle (150) may be thicker than the sensor unit (100). The handle (150) may be too thick to be inserted in to the slot of the device. Thus, the handle (150) may act as a stopper, preventing the sensor unit (100) from being inserted too far into the device. Therefore, the likelihood of damage to the sensor unit (100) or the device due to forceful insertion of the sensor unit (100) is reduced. Furthermore, the handle (150) may therefore act as an indicator that the sensor unit (100) is inserted fully in to the correct position in the device.

In another configuration, the sensor unit (100) may be integral to the exhaled breath collection device (1000). The sensor unit (100) may not be removable from the device as part of normal operation of the device.

With the sensor unit (100) being part of the device it is not necessary to employ a separate device for sensing. Thus, the collection and sampling process is cheaper, easier and faster. Furthermore, with the sensor unit (100) disposed below the condensation surface (21), the cooling and condensation processes are faster than with alternative arrangements.

*Sensor chemistry*

The biosensor (110) may comprise an electrode (120). The electrode (120) is preferably coated with a suitable active agent for detecting the biomarker, dispersed in a conductive polymer. By way of example, for a hydrogen peroxide sensor, potassium ferric ferrocyanide can be dispersed in a conductive polymer. The conductive polymer may be poly(3,4-ethylenedioxythiophene)-poly(styrenesulfonate). The conductive polymer may optionally be stabilised, for example with ethylene glycol and divinyl sulfone.

The active agent dispersed in a conductive polymer may be further dispersed in water to be applied to the electrode (120) to form a coating on the electrode (120). The coating may be applied to the electrode (120) by dropping, printing, spin-coating or similarly depositing the active agent dispersed in a conductive polymer on to the electrode (120).

Preferably, for a hydrogen peroxide sensor, there is at least 0.1 nmol/cm$^2$ of potassium ferric ferrocyanide on the surface of the electrode (120).

The coating, comprising the active agent, can be applied more easily than with traditional electrochemical deposition, thus reducing the manufacturing costs. The coating may also be applied as a more uniform film than in the use of different coating methods and with the use of different conductive polymers. The inclusion of ethylene glycol and divinyl sulfone not only improves mechanical stability of the biosensor (110) but, when sensing hydrogen peroxide, also improves hydrogen peroxide diffusion inside the coating.

*Temperature control*

As illustrated in figure 2, the temperature control unit may comprise a power output (410) configured to power the cooling device (200). The temperature control unit may also comprise a cooling temperature sensor (420) configured to measure the temperature of the cooling device (200). The cooling temperature sensor (420) may be disposed to measure the temperature of the condensation surface (21). The temperature control unit may also comprise a microcontroller (430) configured to generate a control signal according to the temperature measured by cooling temperature sensor (420). The microcontroller (430) may also be configured to send the control signal to the power output (410) to control the power output (410).

The temperature control unit may, for example, comprise a proportional-integral-derivative (PID) controller to reach and consistently maintain the target temperature. The microcontroller (for example Microchip Technology Inc. PIC18f47k40) may send a control signal to control a power output such as an H-bridge (for example Texas Instrument DRV592,). The power can then be fed into the cooling device (200) (for example CUI CP40347) to cool the condensation surface (21). Additionally, a temperature sensor (for example Semitec 103JT-025) may be adopted to measure the temperature of the cooling device (200) or condensation surface (21). The microcontroller can then utilise the temperature data in a control loop algorithm, and the calculated control result can then be sent to the power output as control signal. Once the desired temperature is reached, the control unit can sustain the temperature at that level for the desired duration.

*Variable temperature device*

It was discussed above that for an analyte such as hydrogen peroxide, with a relatively high Henry's law constant, a relatively high target temperature may be set in the exhaled breath collection device (1000) for collecting a sample with adequate concentration of the analyte. Conversely, for a different analyte, such as nitric oxide, which has very low Henry's law constant, the dominant resistance would be in the liquid phase and therefore concentration of nitric oxide in EBC would have two maxima. A first maxima occurs at a high condensation collection temperature at which, due to the lower volume of condensate, concentration of nitric oxide in the EBC would be higher. A second maxima occurs at substantially lower condensation temperature, due to the increasing Henry's law constant with decreasing temperature. At sufficiently low temperature, the liquid phase resistance drops significantly to increase the overall concentration of nitric oxide in exhaled breath condensate.

In order to provide a device which may be used for the analysis of different biomarkers, it is appropriate for the target temperature to be variable. Therefore, the target temperature can be changed according to the biomarker of interest.

In an aspect of the disclosure, the temperature control unit can, in use, control the cooling device (200) such that the target temperature is variable. The temperature control unit may be configured to set the target temperature, wherein the target temperature is variable across the range of from 0 °C to 30 °C. Alternatively, the target temperature may be variable across the range of from 15 °C to 30 °C. The target temperature may be greater than 5 °C and less than or equal to 30 °C, or the target temperature may be greater than 10 °C and less than or equal to 30 °C. Alternatively, the target temperature may be greater than 5 °C and less than or equal to 20 °C, or the target temperature may be greater than 10 °C and less than or equal to 20 °C. That is, the device may be set to operate at any temperature over these ranges.

Temperatures below 0°C may be desirable for some analytes, but can be energy intensive to achieve and maintain in a portable device. Thus, the lower limit on the temperature range is set such that it can be achieved without the device being too bulky to be carried and used by a user at home. The upper limit of 30 °C has been selected by the inventors following their discovery that, for some biomarkers, a higher concentration of analyte in the samples is obtained if the exhaled breath is not cooled to as low a temperature as is commonly applied. For example, as discussed in the example above, for the collection of hydrogen peroxide it is preferable to set a target temperature as defined above. In order to provide a device applicable to a wider range of biomarkers, it is therefore appropriate to increase the range across which the temperature may be set.

The device may comprise means for the user to set the target temperature. The target temperature may be set by a user from within the range across which the target temperature is variable.

The temperature control unit may be configured such that the target temperature corresponds to a selected biomarker. For example, the device may comprise means to select the biomarker to be analysed and there may be a predetermined target temperature set to correspond to the selected biomarker.

For a biomarker with Henry's law solubility constant greater than $1\times10^{-2}$ $mol.m^{-3}.Pa^{-1}$ at 25 °C the target temperature is preferably between 10 and 30 °C. The specific target temperature selected within this range may depend on the biomarker to be analysed.

The device is configured such that the target temperature may be set, either automatically or be manual intervention by the user, with respect to the desired target analyte (i.e., the selected biomarker) to promote the generation of condensate with a high concentration of analyte. The condensation target temperature would depend on the intrinsic properties of the desired target analyte, Henry's law constant and Van't Hoff isotherm. For example hydrogen peroxide would have a preferred condensation target temperature of 20°C and nitric oxide at 0°C.

For example, the Henry's law constant values corresponding to biomarkers may be stored in the memory of the device, or on an external source accessible by the device. The device may comprise means for a user to select the biomarker to be analysed. The temperature control unit may be configured to set the target temperature based on the Henry's law constant value stored for the selected biomarker. The temperature control unit may be configured to set a higher target temperature for a biomarker with a higher Henry's law constant compared to the target temperature for a biomarker with a lower Henry's law constant.

For analyte such as hydrogen peroxide with Henry's law constant of about 820 $mol.m^{-3}.Pa^{-1}$ ($8.3\times10^{4}$ M/atm) and Van't Hoff isotherm (Henry's law temperature dependency) correction 7400 K, the target temperature is preferably between 10 and 25°C, more preferably 20°C for a balance between analyte concentrating and time to condense sufficient EBC.

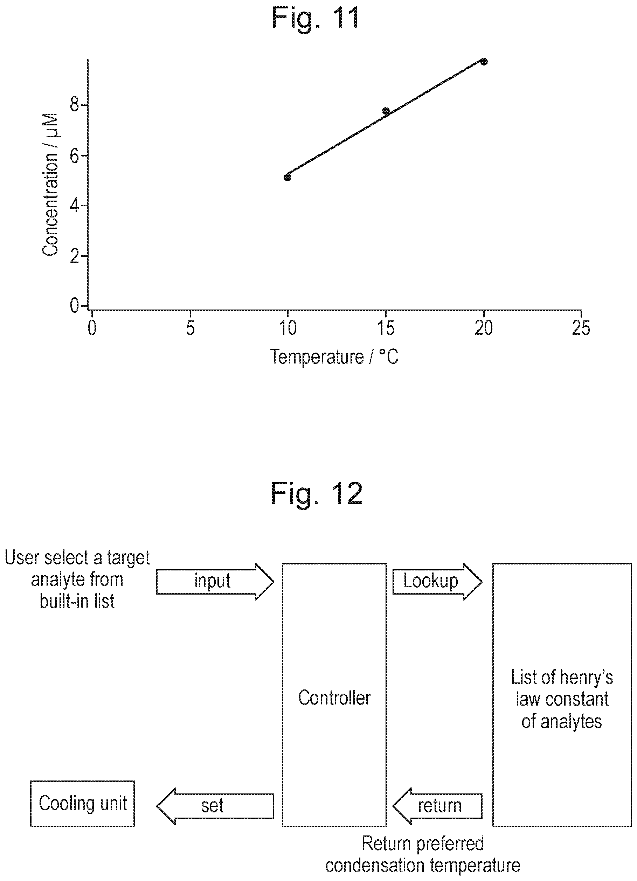

For analytes such as nitric oxide with Henry's law constant of about 1.875 $10^{-5}$ $mol.m^{-3}.Pa^{-1}$ ($1.9\times10^{-3}$ M/atm) and Van't Hoff isotherm correction 1400 K, the target temperature is preferably between -20 and 0°C, more preferably 0°C for a balance between analyte concentrating and device power consumption. Experimental results given in Fig. 11, demonstrate that higher concentration of hydrogen peroxide was collected with higher condensation temperature.

The variable temperature, in a device configured to have a removable sensor unit (100), enables the user to use the same device with multiple different removable/insertable sensor unit (100) which may have different types of biosensor (110) configured to measure different biomarkers in the collected samples. As such, the device is adaptable to different sensing applications associated with the collection and analysis of different biomarkers. This saves the cost associated with having different dedicated devices for each biomarker to be analysed. Furthermore, the sensor unit (100) may be removed without removing any of the other components of the device. This saves cost and is less wasteful than devices requiring a whole cartridge, which may include the sensor unit (100), condensation surface (21) and internal walls of the flow space (10), to be removed and replaced after each use of the device.

*Cleaning system*

The exhaled breath collection device (1000) may be configured such that the condensation surface (21) is removable. The condensation surface (21) may be a surface of a condensation wall (20) of the device. The condensation wall (20) is preferably disposed parallel to a surface of the cooling device (200). The condensation wall (20) may be in contact with the cooling device (200). Alternatively, the condensation wall (20) may be adjacent to, but not in contact with, the cooling device (200).

For a device with an integral condensation surface (21), a ceramic based substrate may be preferable. For a device configured such that the condensation surface (21) is removable, a plastic based substrate may be preferable to reduce cost.

The exhaled breath collection device (1000) may comprise a condensation wall slot wherein the condensation wall (20) is insertable in, and removable from, the condensation wall slot. The condensation wall (20) may comprise a handle (150) configured to protrude from the exhaled breath collection device (1000).

In another configuration, the condensation surface (21) may be integral to the exhaled breath collection device (1000). The condensation surface (21) may not be removable from the device as part of normal operation of the device. For example, the condensation surface (21) may be a surface of the cooling device (200). Alternatively, the condensation surface (21) may be a surface of a wall of the device. The wall may be a separate component not forming part of the cooling device (200). In this arrangement, the wall is preferably parallel to a surface of the cooling device (200). The wall may be in contact with the cooling device (200).

As illustrated in figure 5, the exhaled breath collection device (1000) may further comprise an ultraviolet lamp (510) configured to illuminate the condensation surface (21). The UV lamp (510) may be disposed on an internal wall of the exhaled breath collection device (1000). The internal wall of the exhaled breath collection device (1000) may be opposite to and parallel with the condensation surface (21). The internal wall of the exhaled breath collection device (1000) may be perpendicular to the condensation surface (21). UV light from the UV lamp (510) can aid in cleaning the condensation surface (21) such that exhaled breath condensate remaining on the surface is removed. This is particularly advantageous in the arrangement wherein the condensation surface (21) is not removable from the device. Thus, the device can be reused without subsequent samples becoming contaminated with remaining condensate from previous uses.

The ultraviolet (UV) lamp may be disposed to illuminate the condensation surface (21) when the sensor unit (100) is removed from the exhaled breath collection device (1000). The exhaled breath collection device (1000) may be configured such that the UV light from the UV lamp (510) is obstructed and cannot illuminate the condensation surface (21) when the sensor unit (100) is inserted. The exhaled breath collection device (1000) may be configured such that a rear surface of the sensor unit (100) obstructs the path of UV light from the UV lamp (510) such that the UV light does not illuminate the condensation surface (21), wherein the biosensor (110) is not disposed on the rear surface of the sensor unit (100). This arrangement has the advantage that, during sample collection and analysis, the UV lamp (510) cannot inadvertently interfere with sample collection because the UV lamp (510) is disposed such that it cannot illuminate the biosensor (110) or the condensation surface (21) when the sensor unit (100) is inserted in the exhaled breath collection device (1000).

The temperature control unit may be configured to control the cooling device (200) such that the cooling device (200) reaches a target cleaning temperature. The target cleaning temperature is preferably over 50 °C, and more preferably between 40 °C and 80 °C . The temperature control unit may be configured to control the cooling device (200) such that the condensation surface (21) reaches a target cleaning temperature. The target cleaning temperature may be predetermined or the target cleaning temperature may be set by a user. Heating to the target cleaning temperature can aid in cleaning the condensation surface (21) such that exhaled breath condensate remaining on the surface is removed. This is particularly advantageous in the arrangement wherein the condensation surface (21) is not removable from the device. Thus, the device can be reused without subsequent samples becoming contaminated with remaining condensate formed on the condensation surface (21) during previous uses. In certain arrangements of the device, after each use the sensor unit (100) may be removed and replaced with a new sensor unit (100) for subsequent uses.

In normal operation of the device, with a sensor unit (100) can be inserted, the device can be used to collect and analyse a sample. Once the sample has been analysed, the sensor unit (100) may be removed. The cleaning system may then be activated such that the UV lamp (510) illuminates the condensation surface (21). Furthermore, the cleaning system may be activated such that the temperature is controlled to reach a target cleaning temperature. Thus, the condensation surface (21) is heated. With the condensation surface (21) cleaned, a clean sensor unit (100) may be inserted in the device.

The device may then be used for collection and analysis of a subsequent sample. The type of biosensor (110) and the target temperature may be unchanged for analysis of subsequent samples. Alternatively, the sensor type and target temperature may be changed in order to analyse a different biomarker. The target temperature may be set accordingly for the analyte corresponding to the biomarker of interest. Similarly, the type of biosensor (110) in the sensor unit (100) may be selected accordingly for the analyte corresponding to the biomarker of interest.

*Breath flow measurement and display system*

The exhaled breath collection device (1000) may comprise a breath temperature sensor (610) configured to measure the breath temperature. The breath temperature sensor (610) is preferably disposed downstream of the mouthpiece (300) unit in a direction of flow of breath and upstream of the cooling device (200) in a direction of flow of breath. The breath temperature sensor (610) is more preferably disposed at the downstream end of the mouthpiece (300). The breath temperature sensor (610) may be disposed on the nozzle (310). As illustrated in figure 6, the breath temperature sensor (610) may be disposed on a mouthpiece connector (14), where the mouthpiece connector (14) is configured to attach the mouthpiece (300) to the walls of the device defining the flow space (10). The breath temperature sensor (610) is preferably disposed upstream of the flow space (10). With this configuration the temperature of breath is measured prior to the breath being cooled by proximity to the cooling device (200).

The moisture content in the exhaled breath depends on the temperature of the breath, therefore the temperature of breath entering the device can affect the concentration of analyte collected. Thus, it is beneficial to monitor the temperature of the breath. The device may comprise means to display to the user that the exhaled breath temperature is not in the desired range to collect sufficient concentration of analyte for an accurate measurement.

The exhaled breath collection device (1000) comprises a breath flow sensor (620) configured to measure the breath flow rate of breath introduced to the device through the mouthpiece (300). The breath flow sensor (620) is disposed downstream of the mouthpiece (300) unit in a direction of flow of breath and upstream of the cooling device (200) in a direction of flow of breath. The breath flow sensor (620) is more preferably disposed at the downstream end of the mouthpiece (300). The breath flow sensor (620) may be disposed on the nozzle (310). The breath flow sensor (620) may be disposed on the mouthpiece connector (14). With this configuration the rate of flow of breath is measured prior to the flow impinging on the condensation surface (21).

The exhaled breath collection device (1000) may comprise a breath flow rate indicator (630) configured to display the breath flow rate measured by the flow sensor. The breath flow rate indicator (630) may, for example, comprise a liquid crystal display or a light emitting diode array. The breath flow rate indicator (630) may be configured to display information related to the breath flow rate. For example, the breath flow rate indicator (630) may be configured to display the breath flow rate. The breath flow rate indicator (630) may also be used to display, for example, an indicator to continue to blow into the mouthpiece (300), to blow harder into the mouthpiece (300) or to stop blowing in to the mouthpiece (300).

It is known that analyte concentration can vary with user breathing pattern. For example, it has been previously demonstrated (for example, in Schleiss, M.B., et al., The concentration of hydrogen peroxide in exhaled air depends on expiratory flow rate. European Respiratory Journal, 2000. 16(6): p. 1115-1118) that breath flow rate inversely correlates to hydrogen peroxide concentration in collected exhaled breath condensate. Therefore, the use of a display can aid in collecting a greater concentration of condensate by helping the user to ensure that the breathing flow rate is consistent.

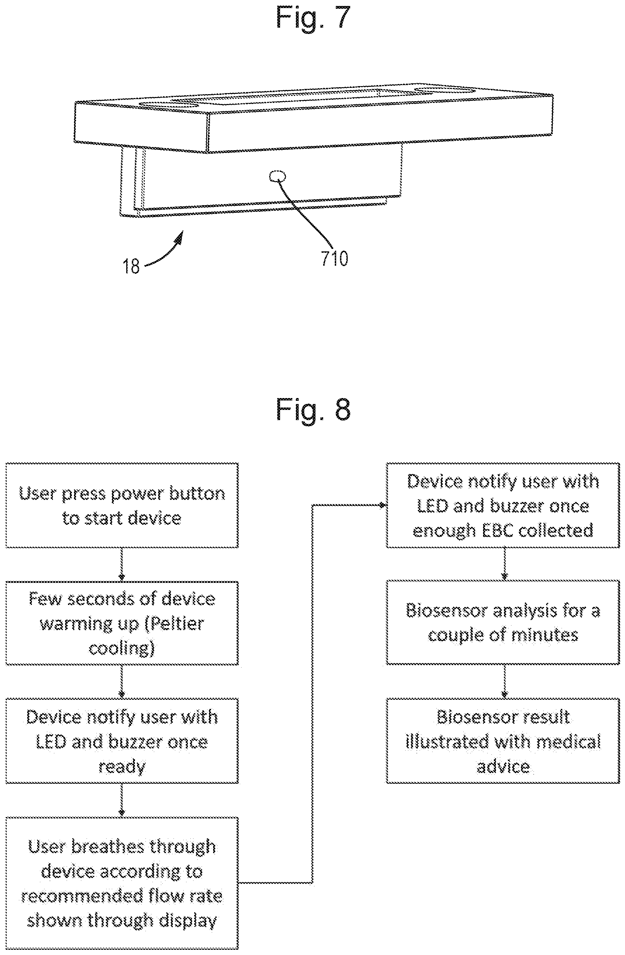

The device may be provided with an exhaust (18), as depicted in figures 1 and 7, configured such that breath from the flow space (10) can leave the device. The exhaust (18) may be disposed above the flow space (10), when the device is held upright, as shown in figure 1. An exit temperature sensor (710) is provided at the device exhaust (18) to measure the temperature of breath leaving the device. The exit temperature sensor (710) may be provided at the device exhaust (18) to measure the temperature of breath leaving the flow space (10). This enables more accurate, detailed analysis of the concentration of biomarker collected.

*Standardisation of condensate collection*

Condensation temperature affects the concentration of the analyte collected, it is therefore useful to measure the condensation parameters in order to determine the amount and/or concentration of condensate collected. This standardization process may be performed through thermofluidic analysis and the process could be split into two parts to simplify the analysis; part 1) condensation of EBC, and part 2) mass transfer of analyte, such as hydrogen peroxide ($H_2O_2$).

**Condensation of EBC**

Fig. 3 illustrates the condensation parameters, including the flow of breath entering and leaving the device, as well as the collected condensate, where $\dot{m}$ is the mass flow rate of breath in kg $s^{-1}$, h is the enthalpy in J $kg^{-1}$, T denotes the temperature in unit K, Φ is the relative humidity, ω is the humidity content or specific humidity in kg $H_2O$(kg air)$^{-1}$, and Q denotes heat.

The process is governed by the amount of moisture entering the device and the amount leaving device. Thus, the amount of moisture/condensate remaining, having been collected in the device, can be established. This may be determined through the use of a humidity sensor, however this has the disadvantage that humidity sensors usually have a slow response and are unsuitable for this application, because breath would be approximately 100% humid entering and leaving the device (relative humidity). The amount of humidity content (specific humidity) can therefore simply be calculated from measuring the temperature of the breath.

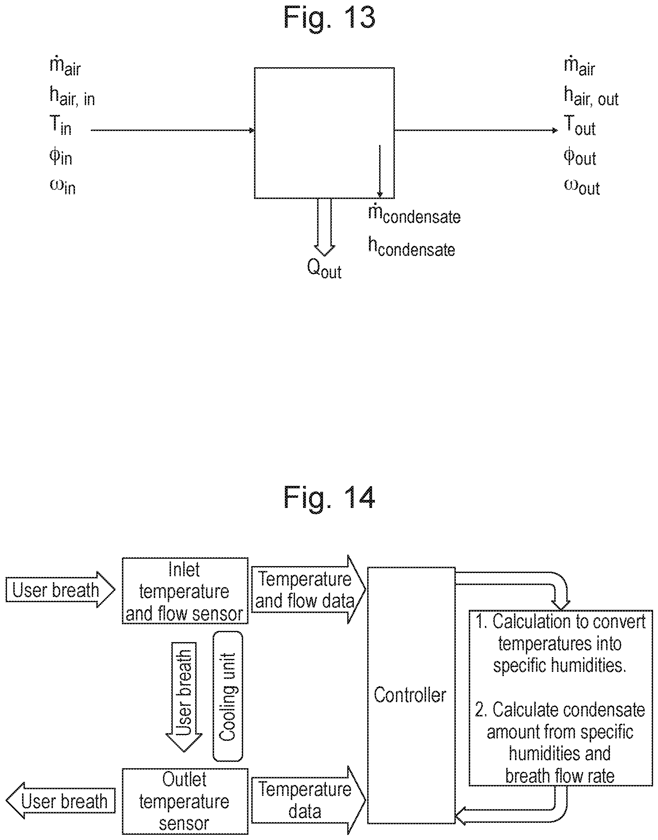

The temperature control unit may be configured to determine the mass of condensate collected in the device (1000), as illustrated in Fig. 14. The temperature data from the breath temperature sensor (610) and exit temperature sensor (710) may be converted into the specific humidities at the inlet and exhaust of the flow space.

The mass of condensate collected may then be determined from the specific humidities and flow rate using the expressions

$$\dot{m} = (\omega_{in} - \omega_{out}) \cdot V_{breath} \tag{2}$$

$$m_{condensate} = \dot{m} \cdot t_{condensation} \tag{3}$$

wherein $\dot{m}$ is the mass flow rate, $m_{condensate}$ is the amount of condensate, $\omega_{in}$ is the specific humidity upstream of the cooling device (200), $\omega_{out}$ is specific humidity at the exhaust (18), $V_{breath}$ is the breath flow rate using flow rate measured by the flow sensor (620), and $t_{condnesation}$ is the condensation time.

Therefore, the amount of condensate collected in the device for each use can be determined using the values measured by the breath temperature sensor (610), the exit temperature sensor (710) and the breath flow sensor (620).

**Mass transfer of hydrogen peroxide**

The two-resistance theory of mass transfer assumes that the rate of mass transfer between two phases is controlled by the rates of diffusion through the phases on each side of the interface. Therefore, the mass transfer can be further split into mass transfer in gas phase and mass transfer in liquid phase.

Assuming phase 1 to be the gas phase (breath) and 2 to be the liquid phase (condensate), also assume that species A is travelling from the gas phase into liquid phase, the mass transfer in both phases can be written as

$$N_{A,G} = k_G \Delta p_A \tag{4}$$

$$N_{A,L} = k_L \Delta C_A \tag{5}$$

where $N_{A,G}$ is the mass transfer in the gas phase, $k_G$ is the convective mass-transfer coefficient in the gas phase, $\Delta p_A$ is the difference in the partial pressure of species A between bulk gas and interface, $N_{A,L}$ is the mass transfer in the liquid phase, $k_L$ is the convective mass-transfer coefficient in the liquid phase, $\Delta C_A$ is the difference in concentration of species A between bulk solution phase and interface.

Interphase concentration/partial pressure is often unknown, it is therefore convenient to have an overall mass-transfer coefficient to describe the interface transfer system. If it is assumed that there is zero resistance across the interface, the concentration of species A at the interface must be in equilibrium. Therefore, Henry's law may be used to find the overall transfer coefficient (where Henry's law is involved). For species A transferring from gas phase to liquid phase the coefficient can be expressed as:

$$\frac{1}{k_{overall}} = \frac{1}{k_G} + \frac{1}{k_H k_L} \tag{6}$$

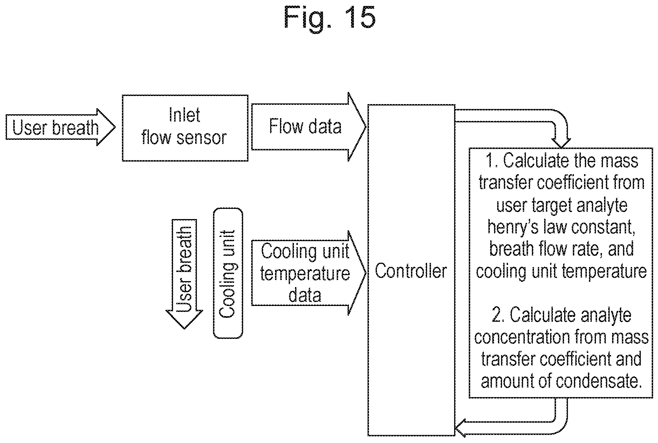

The temperature control unit may be configured to determine the concentration of condensate collected in the device (1000), as illustrated in Fig. 15. The mass transfer coefficient may be determined from the breath flow rate measured by the breath flow sensor (620), the Henry's law constant for the selected biomarker, and temperature of the cooling device, which may be equal to the target temperature or may be measured by the cooling temperature sensor (420). The analyte concentration may then be determined from the mass transfer coefficient and the amount of condensate using two- resistance theory (for example, as described in the textbook Welty, J.R., 2008. Fundamentals of momentum, heat, and mass transfer 5th ed., Hoboken, N.J.; Chichester: Wiley).

The mass transfer depends on flow rate and Henry's law constant and the number of analyte (mol) can be calculated as follows.

$$P_{analyte,out} = P_{analyte,in} \exp\left(\frac{-\overline{k_{overall}} \times A}{V_{breath}}\right) \tag{7}$$

$$\dot{N}_{analyte} = \frac{P_{analyte,in} - P_{analyte,out}}{RT_{breath}} \times V_{breath} \tag{8}$$

P is the partial pressure and $\dot{N}_{analyte}$ is the number of analyte in mol. R is the universal gas constant, A is the area of the condensation surface.

Finally, the predicted concentration can be calculated as

$$[analyte] = \frac{\dot{N}_{analyte} \times \rho_{condensate}}{\dot{m}_{condensate}} \tag{9}$$

Where $\rho_{condensate}$ denotes density of condensate.

Hence using the above expressions, the device may be configured to standardise the concentration of analyte collected with respect to user breath temperature, flow rate and condensation temperature.

**Standardisation method**

In order to determine whether the amount of the analyte in the device indicates that the user has inflammation (i.e., a positive test result), the amount of analyte in the device may be compared to a predetermined limit value known to be indicative of inflammation under standard test conditions.

However, this predetermined limit value is set for standard conditions whereas, in practice, as the device is used there will not necessarily be standard conditions. It is therefore preferably to apply standardisation to "correct" the measured analyte concentration to a standardised equivalent which can be more reasonably compared to the predetermined limit value to show a positive/negative test result.

The device may be configured to apply the standardization process during use of the device. Thus, a correction factor may be applied to determine a corrected amount of analyte, for comparison with the predetermined limit value, and establish a positive or negative test result.

The correction factor may be based on the ratio of the determined amount of condensate collected in the device to a standardised amount of condensate.

$$CF = \frac{[Analyte]_m}{[Analyte]_s} \tag{10}$$

Once the correction factor is determined, it can be used to calculate a corrected amount of condensate as follows.

$$Corrected\ amount\ of\ condensate = \frac{Determined\ amount\ of\ condensate}{CF} \tag{11}$$

The standardised amount of condensate may be determined based on predetermined values of breath temperature downstream of the mouthpiece unit and upstream of the cooling device, breath flow rate downstream of the mouthpiece unit and upstream of the cooling device, temperature of the cooling device and exhaust breath temperature.

One method to determine the correction factor is to use the below equation.

$$(12)\qquad CF = \frac{[Analyte]_m}{[Analyte]_s} = \frac{\left(1-\exp\left(\frac{-\overline{k_{overall,m}}\times A}{V_{breath,m}}\right)\right)(\omega_{in,s}-\omega_{out,s})T_{breath,s}}{\left(1-\exp\left(\frac{-k_{overall,s}\times A}{V_{breath,s}}\right)\right)(\omega_{in,m}-\omega_{out,m})T_{breath,m}}$$

wherein A is the area of the condensation surface, $k_{(overall,m)}$ is the overall mass transfer coefficient; $V_{(breath,m)}$ is the breath flow rate using flow rate measured by the flow sensor; $\omega_{(in,m)}$ is the specific humidity upstream of the cooling device; $\omega_{(out,m)}$ is the specific humidity at the exhaust; $T_{(breath,m)}$ is the exhaust temperature; $k_{(overall,s)}$ is a predetermined standardised overall mass transfer coefficient; $V_{(breath,s)}$ is a predetermined standardised breath flow rate; $\omega_{(in,s)}$ is a predetermined standardised upstream specific humidity; $\omega_{(out,s)}$ is a predetermined standardised exhaust specific humidity; and $T_{(breath,s)}$ is a predetermined standardised exhaust temperature.

Optionally, numerous sample values may be measured over a time t. In this case, the overall correction factor could be calculated as:

$$(13)\qquad CF_{final} = average(CF_{dt}) = \frac{\sum_{t=0}^{t} CF_{dt}}{number\ of\ samples} = \frac{\sum_{t=0}^{t} CF_{dt}}{t*(sample\ rate)}$$

**Example of standardisation method**

A standard parameters of operation were defined as: inlet breath temperature 35 °C, breath volumetric flow rate 3 l/min, target cooling device temperature 20 °C, and outlet breath temperature 29.45 °C. Where the outlet breath temperature was calculated with boundary layer theory for heat transfer for the known geometry of the device.

(14)

$$CF = \frac{[Analyte]_m}{[Analyte]_s} = \frac{\dfrac{\dot{N}_{Analyte,m}\times\rho_{condensate}}{\dot{m}_{condensate,m}}}{\dfrac{\dot{N}_{Analyte,s}\times\rho_{condensate}}{\dot{m}_{condensate,s}}} = \frac{\dfrac{\dot{N}_{Analyte,m}}{\dot{m}_{condensate,m}}}{\dfrac{\dot{N}_{Analyte,s}}{\dot{m}_{condensate,s}}}$$

$$= \frac{\left(1-\exp\left(\frac{-\overline{k_{overall,m}}\times A}{\dot{V}_{breath,m}}\right)\right)\left(\omega_{in,s}-\omega_{out,s}\right)T_{breath,s}}{\left(1-\exp\left(\frac{-\overline{k_{overall,s}}\times A}{\dot{V}_{breath,s}}\right)\right)\left(\omega_{in,m}-\omega_{out,m}\right)T_{breath,m}}$$

Where

(15)

$$\left(1-\exp\left(\frac{-\overline{k_{overall,s}}\times A}{\dot{V}_{breath,s}}\right)\right) = 0.5276$$

$$\left(\omega_{in,s}-\omega_{out,s}\right) = 0.0102$$

$$T_{breath,s} = 302.45\ (K)$$

$$\frac{[Analyte]_m}{[Analyte]_s} = \frac{\left(1-\exp\left(\frac{-\overline{k_{overall,m}}\times A}{\dot{V}_{breath,m}}\right)\right)\times 0.0102\times 302.45}{0.5276\left(\omega_{in,m}-\omega_{out,m}\right)T_{breath,m}}$$

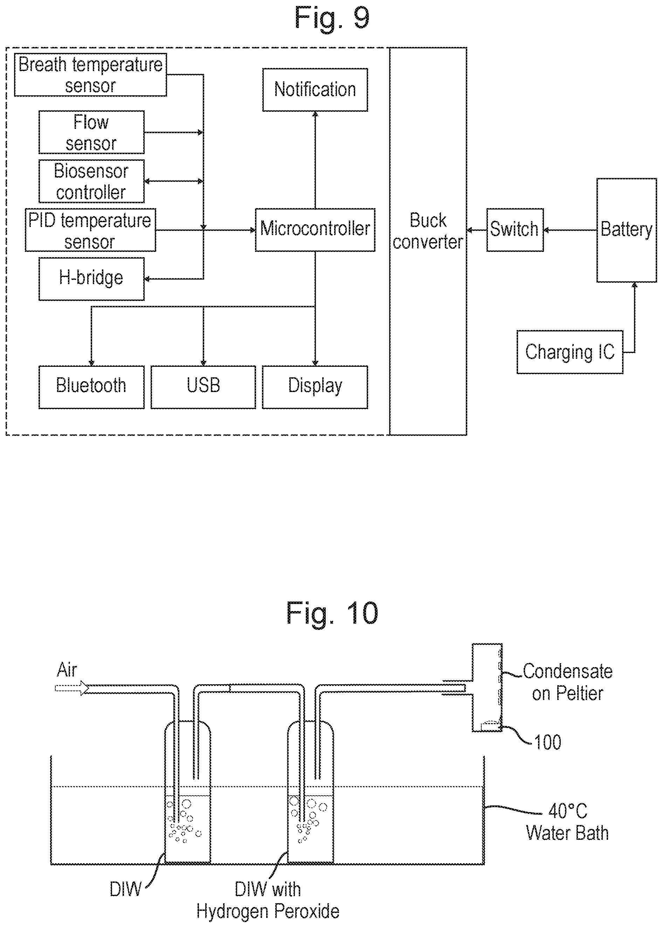

The result of standardisation tested as shown in figure 10 with hydrogen peroxide as analyte with varying condensation temperature, are given in table 1.

Table 1

| Cooling device target temp. (°C) | Standardisation, %($[H_2O_2]_{at\ temperature}$/ $[H_2O_2]_{20°C}$) | Experiment, % ($[H_2O_2]_{at\ temperature}$/ $[H_2O_2]_{20°C}$) |
|---|---|---|
| 10 | 66% | 61% |
| 15 | 79% | 75% |
| 20 | 100% | 100% |

The results in table 1 illustrate that, for instance at a cooling device target condensation temperature of 10°C, the concentration of $H_2O_2$ is 66% of cooling device target condensation temperature of 20°C (standard).

As an example:

1. Under some (standard) circumstances, less than 0.4μM $H_2O_2$ may indicate that the user is healthy whereas equal to or greater than 0.4μM $H_2O_2$ might indicate likely inflammation;
2. The user might collect $H_2O_2$ analyte in EBC at condensation temperature of 10°C;
3. The user breath test with device and disposable sensor might reveal 0.3μM of $H_2O_2$ in user EBC sample.

With no correction, the value 0.3μM of $H_2O_2$ would be considered to indicate that the user is healthy, but with the correction the user would actually has 0.45μM of $H_2O_2$ in user EBC sample and we should consider the user has inflammation.

$$(16)\quad Corrected\ amount\ of\ condensate = \frac{0.3}{66\%} = 0.45$$

Note this example demonstrates effect of condensation temperature with all other variables controlled. In actual application the device correction algorithm can take into account user breathe variables such as breath flow rate, breath inlet and outlet temperature as discussed above.

*Use of the device*

As shown in figure 8, the user may start the device, for example by pressing a power button (11), before waiting for the cooling device (200) to reach the target temperature. The device may then notify the user, for example with an LED and/or buzzer, that the device is ready for breath collection. The user then breathes into the mouthpiece (300). The user may alter their breathing according to information provided on the breath flow rate indicator (630). The device may then notify the user, possibly using the LED and/or buzzer, once a sufficient sample has been collected. The biosensor analysis may then be performed, which may require the user to wait a couple of minutes, depending on the analyte and the sensor. The results of the analysis can then be displayed on the results display unit (17). The device may be configured such that the results can be exported via Bluetooth and/or a USB or alternative connection for further review on a computer.

The target temperature may be predetermined. Alternatively, the target temperature may be variable. If the target temperature is variable, the device may comprise means for the user to set the target temperature after starting the device. The user may set the target temperature by selecting a temperature. The user may optionally select the biomarker of interest from a list of possible biomarkers. The device may be configured to apply a predetermined target temperature corresponding to the selected biomarker.

The sensor unit (100) may be removable. Before using the device, the user may insert the sensor unit (100) in the device. The user may select a sensor unit (100) comprising a biosensor (110) configured to detect the biomarker of interest.

In figure 9, an exemplary diagram of the possible interaction of the various components of the device during use is depicted.

*Effect of cooling temperature - experiment with artificial lung*

The experimental setup used to determine the concentration of hydrogen peroxide detected by an exemplary sensor is illustrated in figure 10.

The experiments were conducted using an exemplary device with the following dimensions:

1. Impinging geometry with condensation surface (21) dimension 30 mm by 30 mm.
2. Nozzle diameter 10 mm at a distance of 10 mm away from the condensation surface (21).

Experimental conditions were as follows:

1. The analyte, in this case hydrogen peroxide, was 1 mM in a Dreschel bottle.
2. The water bath was set to 40 °C to achieve an air exit temperature of 35 °C, such that the "breath" entered the cooling system at approximately 35 °C.
3. The air flow rate was controlled at 3 L/min.
4. Three different Peltier temperatures were adopted: 10 °C, 15 °C, and 20 °C.
5. The air flow was switched on for 5 minutes to allow condensation on the Peltier before collecting the sample for analysis.

The result shown in figure 11 illustrate that the concentration of hydrogen peroxide increased with increased Peltier temperature.

In the above described experiment, the sensor was prepared using the processes as discussed below.

The process of preparing potassium ferric ferrocyanide (PEDOT:PSS-PB) dispersed in poly(3,4-ethylenedioxythiophene)-poly(styrenesulfonate) and dispersed in water was as follows:

1. A step of dissolving 0.215 mmol of ferric chloride in 2 ml of deionised water.
2. A step of dropwise addition of aqueous ferric chloride solution into 5 ml of commercial PEDOT:PSS dispersion and stir for 2 hours.
3. A step of putting the mixture in a centrifuge and washing the mixture with deionised water twice at 8000 rpm for 10 minutes each.
4. A step of dropwise addition of 0.1 M of 20 ml of aqueous potassium ferrocyanide into the washed mixture, and mixing the solution until there is uniform dispersion.
5. A step of stirring the mixture for 15 minutes.
6. A step of putting the mixture in a centrifuge and washing the mixture with deionised water three times at 8000 rpm for 10 minutes each.
7. A step of direct addition of 10 ml of deionised water into product to disperse PEDOT:PSS-PB.

The process of stabilising the PEDOT:PSS solution with ethylene glycol and divinyl sulfone (PEDOT:PSS-PB-EG-DVS) and coating an electrode was as follows:

1. A step of diluting 1 part of PEDOT:PSS-PB with 1 part of deionized water.
2. A step of adding 20% v/v ethylene glycol to PEDOT:PSS-PB.
3. A step of adding 10% v/v divinyl sulfone to PEDOT:PSS-PB.
4. A step of adding an appropriate amount of diluted PEDOT:PSS-PB-EG-DVS onto working electrode, depending on the size of the electrode, using a pipette.
5. A step of leaving the coated electrode to air dry.

The process of applying an inert adsorption layer to the electrode was as follow:

1. A step of preparing the PEDOT:PSS-PB-EG-DVS coated electrode according to the process given above.
2. A step of overlaying an appropriate size of double sided tape.
3. A step of overlaying a polysulfone membrane with 8 $\mu$m pore size.

The above processes are described using the particular values used in the experimental setup shown in figure 10 to generate the results of figure 11. In order to prepare a suitable sensor, the above steps may preferably be applied as described below.

In a preferred process of preparing potassium ferric ferrocyanide (PEDOT:PSS-PB) dispersed in poly(3,4-ethylenedioxythiophene)-poly(styrenesulfonate) and dispersed in water, in step 1, x moles of ferric chloride are dissolved in the deionised water . The value of x may be 0 to 2 times the number of mole of PSS monomer in the PEDOT:PSS solution, but preferably x matches the number of moles of PSS monomer in the PEDOT:PSS solution.

In step 4, y M of aqueous potassium ferrocyanide is added to the washed mixture, where y may preferably be any value from 0 to 1. The value of y may be between the value of x and 100 times the value of x. Preferably, y multiplied by V3 may be slightly greater than x, and preferably V3 is greater than V4, where V4 is preferably approximately equal to V2, and V2 is preferably greater than V1, where:

- the amount of deionised water in step 1 is V1 ml,
- the amount of PEDOT:PSS in step 2 is V2 ml,
- the amount of aqueous potassium ferrocyanide in step 4 is V3 ml, and
- the amount of deionised water in step 7 is V4 ml.

The values of V1 and V3 may, for example, each be between 1 and 20.

In a preferred process of stabilising the PEDOT:PSS solution with ethylene glycol and divinyl sulfone (PEDOT:PSS-PB-EG-DVS), in step 1, 1 part of PEDOT:PSS-PB is preferably diluted with 0 to 10 parts of deionized water, and more preferably 1 part of PEDOT:PSS-PB is diluted with 1 to 3 parts of deionized water, and yet more preferably 1 part of PEDOT:PSS-PB is diluted with 2 parts of deionized water.

In step 2, preferably 5 to 30% v/v ethylene glycol to PEDOT:PSS-PB is added, and more preferably 20% v/v ethylene glycol to PEDOT:PSS-PB is added. In steps 3, preferably 5 to 20% v/v divinyl sulfone to PEDOT:PSS-PB is added, and more preferably 10% v/v divinyl sulfone to PEDOT:PSS-PB is added. Where the concentration, in percentage volume per volume, of divinyl sulfone to PEDOT:PSS-PB is preferably half the concentration, in percentage volume per volume, of ethylene glycol to PEDOT:PSS-PB.

In a preferred process of applying an inert adsorption layer to the electrode, in step 3, the pore size may be 0.01 to 100 μm, and is preferably 0.03 to 100 μm, and is more preferably 0.5 to 10 μm, and yet more preferably the pore size is 5 μm.

Aspects of the present disclosure have been described with particular reference to the examples illustrated. While specific examples are shown in the drawings and are herein described in detail, it should be understood that the drawings and detailed description are not intended to limit the invention to a particular configuration. Variations and modifications may be made to the examples described, provided the result of these modifications is within the scope of the present invention as defined by the claims.

Parts of the device that are depicted in the figures are numbered as follows.

| | |
|---|---|
| 1000 | exhaled breath collection device |
| 10 | flow space |
| 11 | power button |
| 12 | notification unit |
| 13 | heat sink |
| 14 | mouthpiece connector |
| 15 | sensor slot |
| 16 | device electrical contact |
| 17 | results display unit |
| 18 | exhaust |
| 20 | condensation wall |
| 21 | condensation surface |
| 100 | sensor unit |
| 110 | biosensor |
| 120 | electrode |
| 130 | front surface |
| 140 | inert adsorption layer |
| 150 | handle |
| 160 | sensor electrical contact |
| 200 | cooling device |
| 300 | mouthpiece |
| 310 | nozzle |
| 410 | power output |
| 420 | cooling temperature sensor |
| 430 | microcontroller |
| 510 | ultraviolet lamp |
| 610 | breath temperature sensor |
| 620 | breath flow sensor |
| 630 | breath flow rate indicator |
| 710 | exit temperature sensor |

## Claims

1. An exhaled breath collection device (1000) comprising

   a sensor unit (100) configured to measure a biomarker in exhaled breath, wherein the biomarker is hydrogen peroxide ($H_2O_2$),
   a cooling device (200) configured to reduce a temperature of exhaled breath,
   a temperature control unit, and
   a mouthpiece (300) configured to direct exhaled breath towards the cooling device (200);
   wherein the temperature control unit can, in use, control the cooling device (200) to reach a target temperature greater than or equal to 0 °C and less than or equal to 30 °C;
   wherein the exhaled breath collection device (1000) further comprises
   a breath temperature sensor (610) configured to measure the breath temperature **characterised in that** the breath temperature sensor (610) is disposed downstream of the mouthpiece (300) unit in a direction of flow of breath and upstream of the cooling device (200) in a direction of flow of breath;
   and **in that** the exhaled breath collection device (1000) further comprises an exit temperature sensor (710) disposed at a device exhaust (18) to measure the temperature of breath leaving the device; and
   a flow sensor configured to measure the breath flow rate is disposed downstream of the mouthpiece (300) unit in a direction of flow of breath and upstream of the cooling device (200) in a direction of flow of breath;
   wherein the temperature control unit is configured to

   calculate the specific humidity upstream of the cooling device ($\omega_{(in,m)}$) in a direction of flow of breath using breath temperature measured by the breath temperature sensor (610),
   calculate the specific humidity at the exhaust ($\omega_{(out,m)}$) using exhaust temperature ($T_{(breath,m)}$) measured by the exit temperature sensor (710), and
   determine the amount of condensate collected in the device (1000) based on the specific humidity of breath, the specific humidity at the exhaust (18) and the breath flow rate ($V_{(breath,m)}$) using flow rate measured by the flow sensor (620).

2. An exhaled breath collection device (1000) comprising

   a sensor unit (100) configured to measure a biomarker in exhaled breath,
   a cooling device (200) configured to reduce a temperature of exhaled breath,
   a temperature control unit configured to control the cooling device (200) to reach a target temperature, and
   a mouthpiece (300) configured to direct exhaled breath towards the cooling device (200);
   wherein the exhaled breath collection device (1000) further comprises a breath temperature sensor (610) configured to measure the breath temperature,
   **characterised in that** the target temperature is variable across the range of from 0°C to 30°C;
   the breath temperature sensor (610) is disposed downstream of the mouthpiece (300) unit in a direction of flow of breath and upstream of the cooling device (200) in a direction of flow of breath; and the exhaled breath collection device (100) further comprises
   an exit temperature sensor (710) disposed at a device exhaust (18) to measure the temperature of breath leaving the device; and
   a flow sensor configured to measure the breath flow rate is disposed downstream of the mouthpiece (300) unit in a direction of flow of breath and upstream of the cooling device (200) in a direction of flow of breath;
   wherein the temperature control unit is configured to

   calculate the specific humidity upstream of the cooling device ($\omega_{(in,m)}$) in a direction of flow of breath using breath temperature measured by the breath temperature sensor (610),
   calculate the specific humidity at the exhaust ($\omega_{(out,m)}$) using exhaust temperature ($T_{(breath,m)}$) measured by the exit temperature sensor (710), and
   determine the amount of condensate collected in the device (1000) based on the specific humidity of breath, the specific humidity at the exhaust (18) and the breath flow rate ($V_{(breath,m)}$) using flow rate measured by the flow sensor (620).

3. An exhaled breath collection device (1000) according to any of the preceding claims, wherein the cooling device (200) is configured to reduce the temperature of exhaled breath such that exhaled breath condensate is formed, and the sensor unit (100) is configured to measure the biomarker in the exhaled breath condensate.

**4.** An exhaled breath collection device (1000) according to claim 2, wherein the temperature control unit is configured such that the target temperature corresponds to a selected biomarker.

**5.** An exhaled breath collection device (1000) according to claim 4, wherein the temperature control unit is configured to set the target temperature based on the predetermined value of Henry's law constant corresponding to the selected biomarker, optionally wherein the control unit is configured to set the target temperature using an algorithm according to which a higher target temperature is set for a higher value of Henry's law constant.

**6.** An exhaled breath collection device (1000) according to any of the preceding claims, further comprising a condensation surface (21) configured to be cooled by the cooling device (200) and disposed to be exposed to exhaled breath introduced to the device (1000),
optionally wherein either

the condensation surface (21) is disposed in contact with the cooling device (200), or
the condensation surface (21) is a surface of the cooling device (200).

**7.** An exhaled breath collection device (1000) according to claim 6, wherein the condensation surface (21) is disposed perpendicular to the direction of flow of exhaled breath through the mouthpiece (300).

**8.** An exhaled breath collection device (1000) according to any of the preceding claims, wherein the sensor unit (100) comprises an inert adsorption layer (140), optionally wherein the inert adsorption layer (140) comprises

one or more porous polymers, or
one or more porous ceramics.

**9.** An exhaled breath collection device (1000) according to any of the preceding claims, wherein the exhaled breath collection device (1000) is configured such that the sensor unit (100) can be removed.

**10.** An exhaled breath collection device (1000) according to any of the preceding claims, further comprising an electrical contact (16) configured to interface with a corresponding electrical contact (160) of the sensor unit (100).

**11.** An exhaled breath collection device (1000) according to any of the preceding claims, further comprising means to analyse the biomarker measurements taken by the sensor unit (100).

**12.** An exhaled breath collection device (1000) according to any of the preceding claims, wherein the sensor unit (100) comprises an electrode (120) coated with an active agent dispersed in a conductive polymer, optionally wherein the active agent is potassium ferric ferrocyanide, optionally wherein the conductive polymer is poly(3,4-ethylenedioxythiophene)-poly(styrenesulfonate), optionally wherein there is at least 0.1 nmol/cm$^2$ of potassium ferric ferrocyanide on the surface of the electrode (120).

**13.** An exhaled breath collection device (1000) according to any of the preceding claims, wherein the temperature control unit is configured to

determine a corrected amount of condensate based on the determined amount of condensate collected in the device (1000) and a correction factor (CF);
wherein the correction factor (CF) is based on the ratio of the determined amount of condensate collected in the device to a standardised amount of condensate based on predetermined values of breath temperature downstream of the mouthpiece unit and upstream of the cooling device, breath flow rate downstream of the mouthpiece unit and upstream of the cooling device, temperature of the cooling device and exhaust breath temperature.

**14.** An exhaled breath collection device (1000) according to claim 13, wherein the correction factor (CF) is defined by

$$CF = \frac{(1 - \exp\left(\frac{-\overline{k_{overall,m}} \times A}{V_{breath,m}}\right))(\omega_{in,s} - \omega_{out,s})\, T_{breath,s}}{(1 - \exp\left(\frac{-\overline{k_{overall,s}} \times A}{V_{breath,s}}\right))\,(\omega_{in,m} - \omega_{out,m})\, T_{breath,m}}$$

wherein A is the area of the condensation surface (21), $k_{(overall,m)}$ is the overall mass transfer coefficient; $V_{(breath,m)}$ is the breath flow rate using flow rate measured by the flow sensor (620); $\omega_{(in,m)}$ is the specific humidity upstream of the cooling device (200); $\omega_{(out,m)}$ is the specific humidity at the exhaust (18); $T_{(breath,m)}$ is the exhaust temperature; $k_{(overall,s)}$ is a predetermined standardised overall mass transfer coefficient; $V_{(breath,s)}$ is a predetermined standardised breath flow rate; $\omega_{(in,s)}$ is a predetermined standardised upstream specific humidity; $\omega_{(out,s)}$ is a predetermined standardised exhaust specific humidity; and $T_{(breath,s)}$ is a predetermined standardised exhaust temperature.

## Patentansprüche

**1.** Sammelvorrichtung für ausgeatmete Luft (1000), umfassend

eine Sensoreinheit (100), die konfiguriert ist, um einen Biomarker in ausgeatmeter Luft zu messen, wobei der Biomarker Wasserstoffperoxid ($H_2O_2$) ist,
eine Kühlvorrichtung (200), die konfiguriert ist, um eine Temperatur von ausgeatmetem Atem zu senken,
eine Temperatursteuereinheit und
ein Mundstück (300), das konfiguriert ist, um ausgeatmete Luft zu der Kühlvorrichtung (200) zu leiten;
wobei die Temperatursteuereinheit bei Verwendung die Kühlvorrichtung (200) steuern kann, sodass eine Zieltemperatur von über oder gleich 0 °C und unter oder gleich 30 °C erreicht wird;
wobei
die Sammelvorrichtung für ausgeatmete Luft (1000) ferner Folgendes umfasst
einen Atemtemperatursensor (610), der konfiguriert ist, um die Atemtemperatur zu messen, **dadurch gekennzeichnet, dass** der Atemtemperatursensor (610) stromabwärts der Mundstück (300) -Einheit in einer Fließrichtung des Atems und stromaufwärts der Kühlvorrichtung (200) in einer Fließrichtung des Atems angeordnet ist; und dass die Sammelvorrichtung für ausgeatmete Luft (1000) ferner einen Austrittstemperatursensor (710) umfasst, der an einem Vorrichtungsaustritt (18) angeordnet ist, um die Temperatur von Atem zu messen, der die Vorrichtung verlässt; und
ein Fließsensor, der konfiguriert ist, um den Atemdurchsatz zu messen, stromabwärts der Mundstück (300) -Einheit in einer Fließrichtung des Atems und stromaufwärts der Kühlvorrichtung (200) in einer Fließrichtung des Atems angeordnet ist;
wobei die Temperatursteuereinheit für Folgendes konfiguriert ist

Berechnen der spezifischen Feuchtigkeit stromaufwärts der Kühlvorrichtung ($\omega_{(im,m)}$) in einer Fließrichtung des Atems unter Verwendung von Atemtemperatur, gemessen durch den Atemtemperatursensor (610),
Berechnen der spezifischen Feuchtigkeit an dem Austritt ($\omega_{(out,m)}$) unter Verwendung von Austrittstemperatur ($T_{(breath,m)}$), gemessen durch den Austrittstemperatursensor (710), und
Bestimmen der Menge von Kondensat, die in der Vorrichtung (1000) gesammelt wird, basierend auf der spezifischen Feuchtigkeit von Atem, der spezifischen Feuchtigkeit an dem Austritt (18) und dem Atemdurchsatz ($V_{(breath,m)}$) unter Verwendung von Durchsatz, gemessen durch den Fließsensor (620).

**2.** Sammelvorrichtung für ausgeatmete Luft (1000), umfassend

eine Sensoreinheit (100), die konfiguriert ist, um einen Biomarker in ausgeatmeter Luft zu messen,
eine Kühlvorrichtung (200), die konfiguriert ist, um eine Temperatur von ausgeatmeter Luft zu senken,
eine Temperatursteuereinheit, die konfiguriert ist, um die Kühlvorrichtung (200) zu steuern, sodass eine Zieltemperatur erreicht wird, und
ein Mundstück (300), das konfiguriert ist, um ausgeatmete Luft zu der Kühlvorrichtung (200) zu leiten;
wobei die Sammelvorrichtung für ausgeatmete Luft (1000) ferner einen Atemtemperatursensor (610) umfasst, der konfiguriert ist, um die Atemtemperatur zu messen,
**dadurch gekennzeichnet, dass** die Zieltemperatur über den Bereich von 0 °C bis 30 °C hinweg variabel ist; der Atemtemperatursensor (610) stromabwärts der Mundstück (300) - Einheit in einer Fließrichtung des Atems und stromaufwärts der Kühlvorrichtung (200) in einer Fließrichtung des Atems angeordnet ist; und die Sammelvorrichtung für ausgeatmete Luft (100) ferner Folgendes umfasst
einen Austrittstemperatursensor (710), der an einem Vorrichtungsaustritt (18) angeordnet ist, um die Temperatur von Atem zu messen, der die Vorrichtung verlässt; und
ein Fließsensor, der konfiguriert ist, um den Atemdurchsatz zu messen, stromabwärts der Mundstück (300) -Einheit in einer Fließrichtung des Atems und stromaufwärts der Kühlvorrichtung (200) in einer Fließrichtung des Atems angeordnet ist;
wobei die Temperatursteuereinheit für Folgendes konfiguriert ist

Berechnen der spezifischen Feuchtigkeit stromaufwärts der Kühlvorrichtung ($\omega_{(in,m)}$) in einer Fließrichtung des Atems unter Verwendung von Atemtemperatur, gemessen durch den Atemtemperatursensor (610),
Berechnen der spezifischen Feuchtigkeit an dem Austritt ($\omega_{(out,m)}$) unter Verwendung von Austrittstemperatur ($T_{(breath,m)}$), gemessen durch den Austrittstemperatursensor (710), und
Bestimmen der Menge von Kondensat, die in der Vorrichtung (1000) gesammelt wird, basierend auf der spezifischen Feuchtigkeit von Atem, der spezifischen Feuchtigkeit an dem Austritt (18) und dem Atemdurchsatz ($V_{(breath,m)}$) unter Verwendung von Durchsatz, gemessen durch den Fließsensor (620).

**3.** Sammelvorrichtung für ausgeatmete Luft (1000) nach einem der vorhergehenden Ansprüche, wobei die Kühlvorrichtung (200) konfiguriert ist, um die Temperatur von ausgeatmeter Luft zu senken, sodass Kondensat aus ausgeatmeter Luft gebildet wird, und
die Sensoreinheit (100) konfiguriert ist, um den Biomarker in dem Kondensat aus ausgeatmeter Luft zu messen.

**4.** Sammelvorrichtung für ausgeatmete Luft (1000) nach Anspruch 2, wobei die Temperatursteuereinheit konfiguriert ist, sodass die Zieltemperatur einem ausgewählten Biomarker entspricht.

**5.** Sammelvorrichtung für ausgeatmete Luft (1000) nach Anspruch 4, wobei die Temperatursteuereinheit konfiguriert ist, um die Zieltemperatur basierend auf dem vorbestimmten Wert der Henry-Konstante entsprechend dem ausgewählten Biomarker festzulegen, wobei optional die Steuereinheit konfiguriert ist, um die Zieltemperatur unter Verwendung eines Algorithmus festzulegen, gemäß dem eine höhere Zieltemperatur für einen höheren Wert der Henry-Konstante festgelegt wird.

**6.** Sammelvorrichtung für ausgeatmete Luft (1000) nach einem der vorhergehenden Ansprüche, ferner umfassend eine Kondensationsfläche (21), die konfiguriert ist, um durch die Kühlvorrichtung (200) gekühlt zu werden, und angeordnet ist, um ausgeatmeter Luft ausgesetzt zu werden, die der Vorrichtung (1000) zugeführt wird,
wobei optional entweder

die Kondensationsfläche (21) in Kontakt mit der Kühlvorrichtung (200) angeordnet ist oder
die Kondensationsfläche (21) eine Oberfläche der Kühlvorrichtung (200) ist.

**7.** Sammelvorrichtung für ausgeatmete Luft (1000) nach Anspruch 6, wobei die Kondensationsfläche (21) senkrecht zu der Fließrichtung der ausgeatmeten Luft durch das Mundstück (300) angeordnet ist.

**8.** Sammelvorrichtung für ausgeatmete Luft (1000) nach einem der vorhergehenden Ansprüche, wobei die Sensoreinheit (100) eine inerte Adsorptionsschicht (140) umfasst, wobei optional die inerte Adsorptionsschicht (140) Folgendes umfasst

ein oder mehrere poröse Polymere oder
eine oder mehrere poröse Keramiken.

**9.** Sammelvorrichtung für ausgeatmete Luft (1000) nach einem der vorhergehenden Ansprüche, wobei die Sammelvorrichtung für ausgeatmete Luft (1000) konfiguriert ist, sodass die Sensoreinheit (100) entfernt werden kann.

**10.** Sammelvorrichtung für ausgeatmete Luft (1000) nach einem der vorhergehenden Ansprüche, ferner umfassend einen elektrischen Kontakt (16), der konfiguriert ist, um sich mit einem entsprechenden elektrischen Kontakt (160) der Sensoreinheit (100) zu verknüpfen.

**11.** Sammelvorrichtung für ausgeatmete Luft (1000) nach einem der vorhergehenden Ansprüche, ferner umfassend Mittel zum Analysieren der Biomarker-Messungen, die durch die Sensoreinheit (100) genommen werden.

**12.** Sammelvorrichtung für ausgeatmete Luft (1000) nach einem der vorhergehenden Ansprüche, wobei die Sensoreinheit (100) eine Elektrode (120) umfasst, die mit einem Wirkstoff beschichtet ist, der in einem leitfähigen Polymer dispergiert ist, wobei optional der Wirkstoff Kaliumferriferrocyanid ist, wobei optional das leitfähige Polymer Poly(3,4-ethylendioxythiophen)-poly(styrolsulfonat) ist, wobei optional mindestens 0,1 nmol/cm$^2$ Kaliumferriferrocyanid auf der Oberfläche der Elektrode (120) sind.

**13.** Sammelvorrichtung für ausgeatmete Luft (1000) nach einem der vorhergehenden Ansprüche, wobei die Temperatursteuereinheit für Folgendes konfiguriert ist

Bestimmen einer korrigierten Menge von Kondensat basierend auf der bestimmten Menge von Kondensat, die in der Vorrichtung (1000) gesammelt wurde, und einem Korrekturfaktor (CF);
wobei der Korrekturfaktor (CF) auf dem Verhältnis der bestimmten Menge von Kondensat, die in der Vorrichtung gesammelt wurde, zu einer standardisierten Menge von Kondensat basierend auf vorbestimmten Werten von Atemtemperatur stromabwärts der Mundstückeinheit und stromaufwärts der Kühlvorrichtung, Atemdurchsatz stromabwärts der Mundstückeinheit und stromaufwärts der Kühlvorrichtung, und Temperatur der Kühlvorrichtung und Temperatur der ausgeatmeten Luft basiert.

**14.** Sammelvorrichtung für ausgeatmete Luft (1000) nach Anspruch 13, wobei der Korrekturfaktor (CF) definiert ist durch

$$CF = \frac{\left(1 - \exp\left(\frac{-\overline{k_{overall,m}} \times A}{V_{breath,m}}\right)\right)\left(\omega_{in,s} - \omega_{out,s}\right) T_{breath,s}}{\left(1 - \exp\left(\frac{-\overline{k_{overall,s}} \times A}{V_{breath,s}}\right)\right)\left(\omega_{in,m} - \omega_{out,m}\right) T_{breath,m}},$$

wobei A der Bereich der Kondensationsfläche (21) ist, $k_{(overall,m)}$ der gesamte Massentransferkoeffizient ist; $V_{(breath,m)}$ der Atemdurchsatz unter Verwendung von Durchsatz, der durch den Fließsensor (620) gemessen wird, ist; $\omega_{(in,m)}$ die spezifische Feuchtigkeit stromaufwärts der Kühlvorrichtung (200) ist; $\omega_{(out,m)}$ die spezifische Feuchtigkeit am Austritt (18) ist; $T_{(breath,m)}$ die Austrittstemperatur ist; $k_{(overall,s)}$ ein vorbestimmter, standardisierter gesamter Massentransferkoeffizient ist; $V_{(breath,s)}$ ein vorbestimmter, standardisierter Atemdurchsatz ist;$\omega_{(in,s)}$ eine vorbestimmte, standardisierte, stromaufwärtige spezifische Feuchtigkeit ist; $\omega_{(out,s)}$ eine vorbestimmte, standardisierte spezifische Feuchtigkeit am Austritt ist; und $T_{(breath,s)}$ eine vorbestimmte, standardisierte Austrittstemperatur ist.

## Revendications

**1.** Dispositif de collecte de souffle expiré (1000) comprenant

une unité de capteur (100) configurée pour mesurer un biomarqueur dans le souffle expiré, dans lequel le biomarqueur est le peroxyde d'hydrogène ($H_2O_2$),
un dispositif de refroidissement (200) configuré pour réduire une température de souffle expiré,
une unité de commande de température, et
un embout buccal (300) configuré pour diriger le souffle expiré vers le dispositif de refroidissement (200) ;
dans lequel l'unité de commande de température peut, pendant l'utilisation, commander le dispositif de refroidissement (200) pour qu'il atteigne une température cible supérieure ou égale à 0 °C et inférieure ou égale à 30 °C ;
dans lequel le dispositif de collecte de souffle expiré (1000) comprend en outre
un capteur de température de souffle (610) configuré pour mesurer la température de souffle **caractérisé en ce que** le capteur de température de souffle (610) est disposé en aval de l'unité d'embout buccal (300) dans une direction de circulation de souffle et en amont du dispositif de refroidissement (200) dans une direction de circulation de souffle ;
et **en ce que** le dispositif de collecte de souffle expiré (1000) comprend en outre un capteur de température de sortie (710) placé au niveau d'une évacuation de dispositif (18) pour mesurer la température de souffle quittant le dispositif ; et
un capteur de débit configuré pour mesurer le débit de souffle est disposé en aval de l'unité d'embout buccal (300) dans une direction de circulation de souffle et en amont du dispositif de refroidissement (200) dans une direction de circulation de souffle ;
dans lequel l'unité de commande de température est configurée pour

calculer l'humidité spécifique en amont du dispositif de refroidissement ($\omega_{(in,m)}$) dans une direction de circulation de souffle à l'aide d'une température de souffle mesurée par le capteur de température de souffle (610),
calculer l'humidité spécifique au niveau de l'évacuation ($\omega_{(out,m)}$) à l'aide d'une température d'évacuation ($T_{(breath,m)}$) mesurée par le capteur de température de sortie (710), et
déterminer la quantité de condensat collecté dans le dispositif (1000) sur la base de l'humidité spécifique de souffle, de l'humidité spécifique au niveau de l'évacuation (18) et du débit de souffle ($V_{(breath,m)}$) à l'aide du débit mesuré par le capteur de débit (620).

2. Dispositif de collecte de souffle expiré (1000) comprenant

une unité de capteur (100) configurée pour mesurer un biomarqueur dans le souffle expiré,
un dispositif de refroidissement (200) configuré pour réduire une température de souffle expiré,
une unité de commande de température configurée pour commander le dispositif de refroidissement (200) pour qu'il atteigne une température cible, et
un embout buccal (300) configuré pour diriger le souffle expiré vers le dispositif de refroidissement (200) ;
dans lequel le dispositif de collecte de souffle expiré (1000) comprend en outre un capteur de température de souffle (610) configuré pour mesurer la température de souffle,
**caractérisé en ce que** la température cible est variable dans la plage de 0 °C à 30 °C ;
le capteur de température de souffle (610) est disposé en aval de l'unité d'embout buccal (300) dans une direction de circulation de souffle et en amont du dispositif de refroidissement (200) dans une direction de circulation de souffle ; et le dispositif de collecte de souffle expiré (100) comprend en outre
un capteur de température de sortie (710) disposé au niveau d'une évacuation de dispositif (18) pour mesurer la température de souffle quittant le dispositif ; et
un capteur de débit configuré pour mesurer le débit de souffle est disposé en aval de l'unité d'embout buccal (300) dans une direction de circulation de souffle et en amont du dispositif de refroidissement (200) dans une direction de circulation de souffle ;
dans lequel l'unité de commande de température est configurée pour

calculer l'humidité spécifique en amont du dispositif de refroidissement ($\omega_{(in,m)}$) dans une direction de circulation de souffle à l'aide d'une température de souffle mesurée par le capteur de température de souffle (610),
calculer l'humidité spécifique au niveau de l'évacuation ($\omega_{(out,m)}$) à l'aide d'une température d'évacuation ($T_{(breath,m)}$) mesurée par le capteur de température de sortie (710), et

déterminer la quantité de condensat collecté dans le dispositif (1000) sur la base de l'humidité spécifique de souffle, de l'humidité spécifique au niveau de l'évacuation (18) et du débit de souffle ($V_{(breath,m)}$) à l'aide du débit mesuré par le capteur de débit (620).

3. Dispositif de collecte de souffle expiré (1000) selon l'une quelconque des revendications précédentes, dans lequel le dispositif de refroidissement (200) est configuré pour réduire la température de souffle expiré de sorte qu'un condensat de souffle expiré soit formé, et
l'unité de capteur (100) est configurée pour mesurer le biomarqueur dans le condensat de souffle expiré.

4. Dispositif de collecte de souffle expiré (1000) selon la revendication 2, dans lequel l'unité de commande de température est configurée de sorte que la température cible corresponde à un biomarqueur sélectionné.

5. Dispositif de collecte de souffle expiré (1000) selon la revendication 4, dans lequel l'unité de commande de température est configurée pour établir la température cible sur la base de la valeur prédéterminée de la constante de la loi de Henry correspondant au biomarqueur sélectionné, éventuellement dans lequel l'unité de commande est configurée pour régler la température cible à l'aide d'un algorithme selon lequel une température cible plus élevée est établie pour une valeur plus élevée de la constante de la loi de Henry.

6. Dispositif de collecte de souffle expiré (1000) selon l'une quelconque des revendications précédentes, comprenant en outre une surface de condensation (21) configurée pour être refroidie par le dispositif de refroidissement (200) et disposée pour être exposée au souffle expiré introduit dans le dispositif (1000), éventuellement dans lequel soit

la surface de condensation (21) est disposée en contact avec le dispositif de refroidissement (200), soit
la surface de condensation (21) est une surface du dispositif de refroidissement (200).

7. Dispositif de collecte de souffle expiré (1000) selon la revendication 6, dans lequel la surface de condensation (21) est disposée perpendiculairement à la direction de circulation de souffle expiré à travers l'embout buccal (300).

8. Dispositif de collecte de souffle expiré (1000) selon l'une quelconque des revendications précédentes, dans lequel l'unité de capteur (100) comprend une couche d'adsorption inerte (140), éventuellement dans lequel la couche d'adsorption inerte (140) comprend

un ou plusieurs polymères poreux, ou
une ou plusieurs céramiques poreuses.

9. Dispositif de collecte de souffle expiré (1000) selon l'une quelconque des revendications précédentes, dans lequel le dispositif de collecte de souffle expiré (1000) est configuré de sorte que l'unité de capteur (100) puisse être retirée.

10. Dispositif de collecte de souffle expiré (1000) selon l'une quelconque des revendications précédentes, comprenant en outre un contact électrique (16) configuré pour s'interfacer avec un contact électrique (160) correspondant de l'unité de capteur (100).

11. Dispositif de collecte de souffle expiré (1000) selon l'une quelconque des revendications précédentes, comprenant en outre des moyens d'analyse des mesures de biomarqueur prises par l'unité de capteur (100).

12. Dispositif de collecte de souffle expiré (1000) selon l'une quelconque des revendications précédentes, dans lequel l'unité de capteur (100) comprend une électrode (120) revêtue d'un agent actif dispersé dans un polymère conducteur, éventuellement dans lequel l'agent actif est le ferrocyanure ferrique de potassium, éventuellement dans lequel le polymère conducteur est le poly(3,4-éthylènedioxythiophène)-poly(styrènesulfonate), éventuellement dans lequel il y a au moins 0,1 nmol/cm$^2$ de ferrocyanure ferrique de potassium sur la surface de l'électrode (120).

13. Dispositif de collecte de souffle expiré (1000) selon l'une quelconque des revendications précédentes, dans lequel l'unité de commande de température est configurée pour

déterminer une quantité corrigée de condensat sur la base de la quantité déterminée de condensat collecté dans le dispositif (1000) et d'un facteur de correction (CF) ;
dans lequel le facteur de correction (CF) est basé sur le rapport entre la quantité déterminée de condensat collecté dans le dispositif et une quantité normalisée de condensat sur la base de valeurs prédéterminées de température de souffle en aval de l'unité d'embout buccal et en amont du dispositif de refroidissement, de débit de souffle en aval de l'unité d'embout buccal et en amont du dispositif de refroidissement, de température du dispositif de refroidissement et de température de souffle d'évacuation.

14. Dispositif de collecte de souffle expiré (1000) selon la revendication 13, dans lequel le facteur de correction (CF) est défini par

$$CF = \frac{\left(1 - \exp\left(\frac{-\overline{k_{overall,m}} \times A}{V_{breath,m}}\right)\right)\left(\omega_{in,s} - \omega_{out,s}\right) T_{breath,s}}{\left(1 - \exp\left(\frac{-\overline{k_{overall,s}} \times A}{V_{breath,s}}\right)\right)\left(\omega_{in,m} - \omega_{out,m}\right) T_{breath,m}}$$

où A est l'aire de la surface de condensation (21), $k_{(overall,m)}$ est le coefficient de transfert de masse global ; $V_{(breath,m)}$ est le débit de souffle à l'aide du débit mesuré par le capteur de débit (620) ; $\omega_{(in,m)}$ est l'humidité spécifique en amont du dispositif de refroidissement (200) ; $\omega_{(out,m)}$ est l'humidité spécifique au niveau de l'évacuation (18) ;

$T_{(breath,m)}$ est la température d'évacuation ; $k_{(overall,s)}$ est un coefficient de transfert de masse global normalisé prédéterminé ; $V_{(breath,s)}$ est un débit de souffle normalisé prédéterminé; $\omega_{(in,s)}$ est une humidité spécifique en amont normalisée prédéterminée ; $\omega_{(out,s)}$ est une humidité spécifique d'évacuation normalisée prédéterminée ; et $T_{(breath,s)}$ est une température d'évacuation normalisée prédéterminée.

Fig. 1

1000
630, 17
18
610, 620
13
420
200
300
100
12
11

Fig. 2

Cooling device
200
Temperature
Measurement
Cooling temperature sensor
420
Temperature
signal
Power
Power output
410
Microcontroller
430
Control
signal

Fig. 3

21
10
310
Breathe
300
20

Fig. 4

150
140
110
120
130
160

Fig. 5

16
140
510
15

Fig. 6

14
610
620

Fig. 7

18
710

Fig. 8

User press power button to start device
Few seconds of device warming up (Peltier cooling)
Device notify user with LED and buzzer once ready
User breathes through device according to recommended flow rate shown through display
Device notify user with LED and buzzer once enough EBC collected
Biosensor analysis for a couple of minutes
Biosensor result illustrated with medical advice

Fig. 9

Breath temperature sensor
Flow sensor
Biosensor controller
PID temperature sensor
H-bridge
Notification
Microcontroller
Bluetooth
USB
Display
Buck converter
Switch
Battery
Charging IC

Fig. 10

Air
Condensate on Peltier
100
40°C Water Bath
DIW
DIW with Hydrogen Peroxide

Fig. 11

Concentration / µM
8
6
4
2
0
0
5
10
15
20
25
Temperature / °C

Fig. 12

User select a target analyte from built-in list
input
Controller
Lookup
List of henry's law constant of analytes
Cooling unit
set
return
Return preferred condensation temperature

Fig. 13

$\dot{m}_{air}$
$h_{air, in}$
$T_{in}$
$\varphi_{in}$
$\omega_{in}$
$\dot{m}_{air}$
$h_{air, out}$
$T_{out}$
$\varphi_{out}$
$\omega_{out}$
$\dot{m}_{condensate}$
$h_{condensate}$
$Q_{out}$


Fig. 14

User breath
Inlet temperature and flow sensor
Temperature and flow data
Controller
1. Calculation to convert temperatures into specific humidities.
2. Calculate condensate amount from specific humidities and breath flow rate
User breath
Cooling unit
User breath
Outlet temperature sensor
Temperature data

Fig. 15

User breath
Inlet flow sensor
Flow data
User breath
Cooling unit
Cooling unit temperature data
Controller
1. Calculate the mass transfer coefficient from user target analyte henry's law constant, breath flow rate, and cooling unit temperature
2. Calculate analyte concentration from mass transfer coefficient and amount of condensate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018172761 A1
- US 2008183094 A
- WO 2008022183 A1


### Non-patent literature cited in the description


- **GOLDONI MATTED et al.** Influence of condensation temperature or selected exhaled breath parameters. *BMC Pulmonary Medicine,* 01 September 2005, vol. 5 (1
- **SCHLEISS, M.B. et al.** The concentration of hydrogen peroxide in exhaled air depends on expiratory flow rate. *European Respiratory Journal,* 2000, vol. 16 (6), 1115-1118
- **WELTY, J.R.** Fundamentals of momentum, heat, and mass transfer. Wiley, 2008